# EUROPEAN PATENT APPLICATION

(11) **EP 4 613 202 A1**
(43) Date of publication of application: **10.09.2025**
(21) Application number: 23891837.9
(22) Date of filing: 19.10.2023
(51) Int. Cl.: A61B 6/00, G06V 40/20, G06N 3/08, A61B 6/04, G06N 20/00

(54) **X-RAY IMAGING DEVICE COMPRISING CAMERA, AND OPERATION METHOD THEREFOR**

(30) Priority: 14.11.2022 KR 20220152023; 14.11.2022 KR 20220152024; 24.02.2023 KR 20230025288
(71) Applicant: Samsung Electronics Co., Ltd., Suwon-si, Gyeonggi-do 16677 (KR)
(72) Inventor: KWON, Jaehyun, Suwon-si, Gyeonggi-do 16677 (KR); JO, Hyunhee, Suwon-si, Gyeonggi-do 16677 (KR); MOON, Heeyeon, Suwon-si, Gyeonggi-do 16677 (KR); YANG, Jongho, Suwon-si, Gyeonggi-do 16677 (KR); OH, Hyunhwa, Suwon-si, Gyeonggi-do 16677 (KR); I, Gitae, Suwon-si, Gyeonggi-do 16677 (KR); LEE, Hyunjung, Suwon-si, Gyeonggi-do 16677 (KR)
(74) Representative: Walaski, Jan Filip
(86) International application number: PCT/KR2023/016251
(87) International publication number: WO 2024/106770

(57) **Abstract**

Provided are an X-ray imaging device including a camera and a method of operating the same. The X-ray imaging device according to an embodiment of the present disclosure may obtain an object image by photographing an object with a camera, detect a motion of the object by analyzing the object image using an artificial intelligence (AI) model, and output a notification signal notifying the user of a result of the detecting of the motion of the object. An X-ray imaging device according to an embodiment of the present disclosure may obtain an object image by photographing an object with a camera, obtain a plurality of divided imaging areas for stitching imaging before X-raying by using an artificial intelligence model, and display a plurality of guidelines representing top, bottom, left and right boundaries of a plurality of divided imaging areas.

## Description

### Technical Field

The present disclosure relates to an X-ray imaging device including a camera and a method of operating the same. More particularly, the present disclosure relates to an X-ray imaging device for detecting a motion of an object by using an image obtained by photographing the object with a camera. The present disclosure relates to an X-ray imaging device for performing stitching X-raying by using an image of an object captured with a camera.

### Background Art

In recent years, X-ray imaging devices equipped with a camera and automating setting, moving, patient position and status checking, etc., of the X-ray imaging device by using image data of an object (e.g., a patient) obtained through the camera have been distributed and used. Specifically, the X-ray imaging device including a camera may recognize, from an image obtained through the camera, a position of the patient, posture information, X-ray detection active areas, a location of an automatic exposure control (AEC) chamber, or the like. The X-ray imaging device including a camera has a technical effect of reducing the user's operation time as compared to the traditional X-ray imaging device having no camera.

By using an image of an object (e.g., a patient), the X-ray imaging device including the camera may detect a motion of the patient. The traditional X-ray imaging device detects a motion of the patient by attaching a fiducial element onto a certain body portion of the patient and analyzing a displacement of the fiducial element from an image obtained by photographing the patient with the fiducial element attached thereto. The conventional art does not work when there is no fiducial element to be attached to a certain portion of the patient, and may detect a motion of the patient only in a procedure for obtaining successive X-ray images. The conventional art has a limitation in that it is unable to prevent an abnormal image from being obtained by detecting a motion of the patient before taking X-ray images.

Recently, a technology that enables more precise and rapid patient recognition by introducing an artificial intelligence (AI) technology to the X-ray imaging device is being available. An Al system is a computer system that embodies human-level intelligence allowing a machine to learn and make decisions by itself, and that the more it is used the better the recognition rate is. AI technologies include a machine learning technology that uses an algorithm for self-classifying/self-learning features of input data, or elemental technologies that use a deep learning algorithm to simulate such a function as perception, determination, or the like of a human brain.

### Disclosure of Invention

### Solution to Problem

The present disclosure provides an X-ray imaging device for detecting a motion of an object. According to an embodiment of the present disclosure, an X-ray imaging device may include an X-ray irradiator configured to generate X-rays and irradiate the X-rays to an object, an X-ray detector configured to detect X-rays irradiated by the X-ray irradiator and transmitted through the object, a camera configured to obtain an object image by photographing the object positioned in front of the X-ray detector, a display, and at least one processor. The at least one processor may be configured to detect a motion of the object from the object image by analyzing the object image by using an artificial intelligence (AI) model. The at least one processor may be configured to output a notification signal on the display to notify a user of a result of the detecting of the motion of the object.

The present disclosure provides a method of operating an X-ray imaging device. According to an embodiment of the present disclosure, a method of operating an X-ray imaging device may include obtaining image data of an object by photographing the object with a camera. According to an embodiment of the present disclosure, the method of operating an X-ray imaging device may include detecting a motion of the object from the image data by analyzing the image data by using an Al model. According to an embodiment of the present disclosure, the method of operating an X-ray imaging device may include outputting a notification signal to notify a user of a result of the detecting of the motion of the object.

The present disclosure provides an X-ray imaging device for performing stitching X-raying. According to an embodiment of the present disclosure, an X-ray imaging device may include an X-ray detector configured to detect X-rays irradiated by the X-ray irradiator and transmitted through the object, a camera configured to obtain an object image by photographing the object positioned in front of the X-ray detector, a display, and at least one processor. The at least one processor may be configured to input the object image to a trained Al model, and obtain a plurality of divided imaging areas for stitching X-raying the object through inferencing using an Al model. The at least one processor may be configured to display, on the display, a plurality of guidelines representing top, bottom, and left and right boundaries of the plurality of divided imaging areas.

### Brief Description of Drawings

The present disclosure may be readily understood by combinations of the following detailed descriptions and the accompanying drawings, and reference numerals refer to structural elements.
FIG. 1 is an exterior view illustrating a configuration of an X-ray imaging device, according to an embodiment of the present disclosure.
FIG. 2 is a perspective view of an X-ray detector, according to an embodiment of the present disclosure.
FIG. 3 illustrates an X-ray imaging device including a mobile X-ray detector, according to an embodiment of the present disclosure.
FIG. 4 is a conceptual diagram for describing an operation of an X-ray imaging device for detecting a motion of an object from an image obtained through a camera, according to an embodiment of the present disclosure.
FIG. 5 is a block diagram illustrating components of an X-ray imaging device, according to an embodiment of the present disclosure.
FIG. 6 is a flowchart illustrating a method by which an X-ray imaging device detects a motion of an object from an image obtained through a camera, according to an embodiment of the present disclosure.
FIG. 7 is a diagram for describing an operation of an X-ray imaging device for detecting a motion of an object by comparing a reference image with a subsequent image frame, according to an embodiment of the present disclosure.
FIG. 8 is a flowchart illustrating a method by which an X-ray imaging device detects a motion of an object by using a machine learning algorithm, according to an embodiment of the present disclosure.
FIG. 9 is a flowchart illustrating a method by which an X-ray imaging device detects a motion of an object by using a pre-trained deep neural network model, according to an embodiment of the present disclosure.
FIG. 10 is a conceptual diagram for describing an operation of an X-ray imaging device for detecting a motion of an object by using a pre-trained deep neural network model, according to an embodiment of the present disclosure.
FIG. 11 is a diagram illustrating an operation of an X-ray imaging device for detecting positioning of an object by using a depth measuring device, according to an embodiment of the present disclosure.
FIG. 12 is a block diagram illustrating components of an X-ray imaging device and a workstation, according to an embodiment of the present disclosure.
FIG. 13 is a conceptual diagram for describing an operation of the X-ray imaging device for displaying divided imaging areas for stitching X-raying on an image obtained through a camera, according to an embodiment of the present disclosure.
FIG. 14 is a block diagram illustrating components of an X-ray imaging device, according to an embodiment of the present disclosure.
FIG. 15 is a flowchart illustrating a method by which an X-ray imaging device obtains divided imaging areas for stitching X-raying on an image obtained through a camera and displays a graphical user interface (UI) representing the divided imaging areas, according to an embodiment of the present disclosure.
FIG. 16 is a diagram illustrating an operation of an X-ray imaging device for determining divided imaging areas according to an imaging protocol and displaying a graphical UI representing the determined divided imaging areas, according to an embodiment of the present disclosure.
FIG. 17A is a diagram illustrating an operation of an X-ray imaging device for changing at least one of location, size and shape of a divided imaging area based on a user input, according to an embodiment of the present disclosure.
FIG. 17B is a diagram illustrating an operation of an X-ray imaging device for changing at least one of location, size and shape of a divided imaging area based on a user input, according to an embodiment of the present disclosure.
FIG. 18 is a diagram illustrating an operation of an X-ray imaging device for determining a margin of a divided imaging area based on a user input, according to an embodiment of the present disclosure.
FIG. 19 is a diagram illustrating an operation of an X-ray imaging device for detecting positioning of an object by using a depth measuring device, according to an embodiment of the present disclosure.
FIG. 20 is a block diagram illustrating components of an X-ray imaging device and a workstation, according to an embodiment of the present disclosure.

### Mode for the Invention

The terms are selected from among common terms widely used at present, taking into account principles of the present disclosure, which may however depend on intentions of those of ordinary skill in the art, judicial precedents, emergence of new technologies, and the like. Some terms as herein used are selected at the applicant's discretion, in which case, the terms will be explained below in detail in connection with embodiments. Therefore, the terms should be defined based on their meanings and descriptions throughout the present disclosure.

As used herein, the singular forms "a", "an" and "the" are intended to include the plural forms as well, unless the context clearly indicates otherwise. All terms including technical and scientific terms used herein have the same meaning as commonly understood by one of ordinary skill in the art to which this disclosure belongs.

The term "include (or including)" or "comprise (or comprising)" is inclusive or open-ended and does not exclude additional, unrecited elements or method steps. The terms "unit", "module", "block", etc., as used herein each represent a unit for handling at least one function or operation, and may be implemented in hardware, software, or a combination thereof.

In the present disclosure, the expression "configured to" as herein used may be interchangeably used with "suitable for", "having the capacity to", "designed to", "adapted to", "made to", or "capable of" according to the given situation. The expression "configured to" may not necessarily mean "specifically designed to" in terms of hardware. For example, in some situations, an expression "a system configured to do something" may refer to "an entity able to do something in cooperation with" another device or parts. For example, "a processor configured to perform A, B and C functions" may refer to a dedicated processor, e.g., an embedded processor for performing A, B and C functions, or a general purpose processor, e.g., a Central Processing Unit (CPU) or an application processor that may perform A, B and C functions by executing one or more software programs stored in a memory.

When the term "connected" or "coupled" is used, a component may be directly connected or coupled to another component. However, unless otherwise defined, it is also understood that the component may be indirectly connected or coupled to the other component via another new component.

In the present disclosure, the term 'object' refers to a target to be imaged, including a human, an animal or a part thereof. For example, the object may include a patient, a portion (e.g., organ) of the patient's body or a phantom.

In the present disclosure, 'X-ray' is an electromagnetic wave with a wavelength of 0.01 to 100 angstrom (Å), which has a property of penetrating objects, so it may be generally and widely used in medical equipment that obtains images of the inside of a living body or non-destructive testing equipment in general industry.

In the present disclosure, an X-ray imaging device is a medical imaging device for obtaining an X-ray image of an internal structure of an object (e.g., a patient's body) by transmitting X-rays through the object. The X-ray device is easy to use as compared to other medical imaging devices including an MRI device, a CT device, etc., and may obtain medical images of objects within a short time. Hence, the X-ray device is widely used in simple chest imaging, simple abdominal imaging, simple skeletal imaging, simple sinus imaging, simple neck soft tissue imaging and mammography.

In the present disclosure, the term 'image' or 'object image' may refer to data comprised of discrete image elements (e.g., pixels of a two-dimensional (2D) image). In the present disclosure, the term 'image' or 'object image' refers to an image obtained by a camera having a general image sensor (e.g., CMOS or CCD) In the present disclosure, the term 'image' or 'object image' refers to a different one from an X-ray image obtained by image processing that detects, by an X-ray detector, X-rays transmitted through an object and converts the X-rays to an electric signal.

Functions related to artificial intelligence (AI) in the present disclosure are operated through a processor and a memory. The processor may be provided in plural. The one or more processors may include a universal processor such as a central processing unit (CPU), an application processor (AP), a digital signal processor (DSP), etc., a graphic processing unit (GPU), a vision processing unit (VPU), etc., or a dedicated artificial intelligence (AI) processor such as a neural processing unit (NPU). The one or more processors may control processing of input data according to a predefined operation rule or an Al model stored in the memory. When the one or more processors are the dedicated Al processors, they may be designed in a hardware structure that is specialized for processing a particular Al model.

The predefined operation rule or the Al model may be made by learning. Specifically, the Al model being made by learning refers to the predefined operation rule or the Al model established to perform a desired feature (or an object) being made when a basic Al model is trained by a learning algorithm with a lot of training data. Such learning may be performed by a device itself in which Al is performed according to the present disclosure, or by a separate server and/or system. Examples of the learning algorithm may include supervised learning, unsupervised learning, semi-supervised learning, or reinforcement learning, but are not limited thereto.

In the present disclosure, the Al model may be made up of a plurality of neural network layers. Each of the plurality of neural network layers may have a plurality of weight values, and perform neural network operation through operation between an operation result of the previous layer and the plurality of weight values. The plurality of weight values owned by the plurality of neural network layers may be optimized by learning results of the Al model. For example, the plurality of weight values may be updated to reduce or minimize a loss value or a cost value obtained by the Al model during a training procedure. The artificial neural network model may include a deep neural network (DNN), for example, a convolutional neural network (CNN), a recurrent neural network (RNN), a restricted Boltzmann machine (RBM), a deep belief network (DBN), a bidirectional recurrent deep neural network (BRDNN), or a deep Q-network, but is not limited thereto.

An embodiment of the present disclosure will now be described in detail with reference to accompanying drawings so as to be readily practiced by those of ordinary skill in the art. However, the embodiments of the present disclosure may be implemented in many different forms, and not limited thereto as will be discussed herein.

Embodiments of the present disclosure will now be described in detail with reference to accompanying drawings.

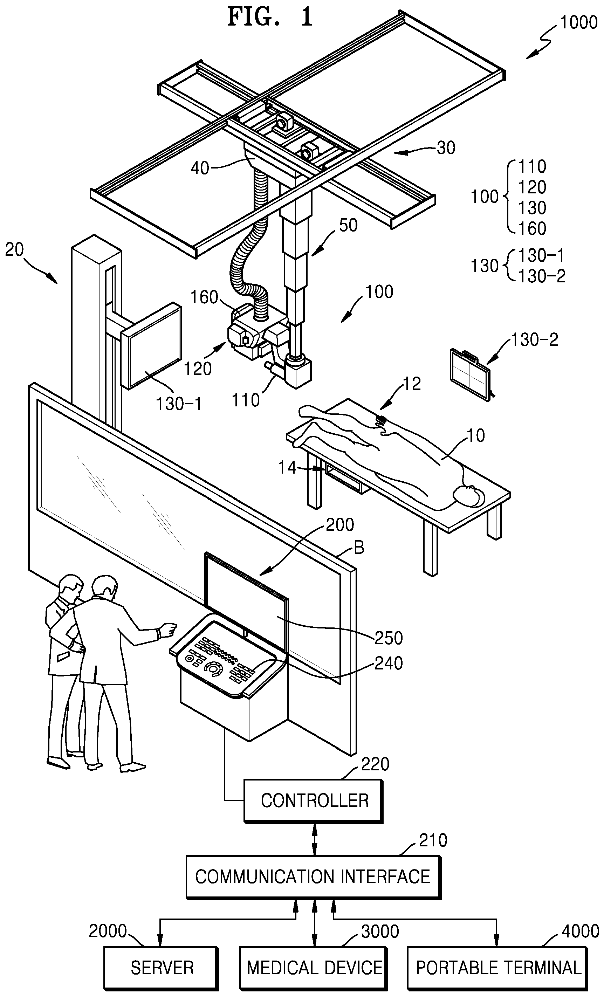

FIG. 1 is an exterior view illustrating a configuration of an X-ray system 1000, according to an embodiment. In FIG. 1, a room X-ray imaging device (Room DR) will be described as an example.

Referring to FIG. 1, the X-ray system 1000 may include an X-ray imaging device 100 and a workstation 200. The X-ray imaging device 100 may include a camera 110 configured to obtain an object image by photographing an object 10, an X-ray irradiator 120 configured to generate and irradiate X-rays to the object 10, an X-ray detector 130 configured to detect X-rays that has been transmitted through the object, and a user input interface 160. In FIG. 1, only essential components for describing operations of the X-ray imaging device 100 are shown, and components of the X-ray imaging device 100 of the present disclosure are not limited to those illustrated in FIG. 1. The workstation 200 may perform data communication with the X-ray imaging device 100, and provide information for the user in response to receiving a command from the user. Furthermore, the X-ray system 1000 may further include a controller 220 for controlling the X-ray system 1000 according to a command input through the workstation, and a communication interface 210 for communicating with an external device. Some or all of the components of the communication interface 210 and the controller 220 may be included in the workstation 200 or provided separately from the workstation 200.

The X-ray irradiator 120 may be equipped with an X-ray source for generating X-rays and a collimator for controlling an irradiation area of X-rays generated from the X-ray source.

A guide rail 30 may be installed on the ceiling of an examination room where the X-ray system 1000 is placed; the X-ray irradiator 120 may be moved to a position corresponding to the object 10 by connecting the X-ray irradiator 120 to a mobile carriage 40 that moves along the guide rail 30; the mobile carriage 40 and the X-ray irradiator 120 may be connected through a foldable post frame 50 to adjust the height of the X-ray irradiator 120.

An input interface 240 for receiving commands from the user and an output interface 250 for displaying information may be arranged on the workstation 200.

The input interface 240 may receive commands for controlling imaging protocol, imaging condition, imaging timing, positioning control over the X-ray irradiator 120, etc. In an embodiment of the present disclosure, the input interface 240 may include a keyboard, a mouse, a touch screen, a voice recognizer, etc.

The output interface 250 may display a screen for guiding user inputs, an X-ray image, a screen indicating a state of the X-ray system 1000, etc. In an embodiment of the present disclosure, the output interface 250 may include a display.

The controller 220 may control imaging timing, imaging condition, etc., of the X-ray irradiator 120 according to a command input from the user, and generate an X-ray image by using image data received from the X-ray detector 130. The controller 220 may also control locations or postures of an installation part 14 or 24 where the X-ray irradiator 120 or the X-ray detector 130 is installed, according to the imaging protocol and the position of the object 10.

The controller 220 may include a memory for storing a program for carrying out the aforementioned and following operations, and a processor for executing the program. The controller 220 may include a single processor or a plurality of processors, and in the latter case, the plurality of processors may be integrated in a single chip or may be physically separated.

The X-ray system 1000 may be connected to an external device (e.g., an external server 2000, a medical device 3000 and a portable terminal 4000 (e.g., a smartphone, a tablet PC, a wearable device, etc.)) through the communication interface 210 to transmit or receive data.

The communication interface 210 may include one or more components that enable communication with the external device, and include, for example, at least one of a short-range communication module, a wired communication module, and a wireless communication module.

The communication interface 210 may receive a control signal from the external device and may also send the received control signal to the controller 220 in order for the controller 220 to control the X-ray system 1000 according to the received control signal.

The processor 220 may also control the external device based on a control signal of the controller 220 by transmitting the control signal to the external device through the communication interface 210. For example, the external device may process data of the external device according to the control signal of the controller 220 received through the communication interface 210.

The communication interface 210 may further include an internal communication module that enables communication between the components of the X-ray system 1000. A program to control the X-ray system 1000 may be installed in the external device, and the program may include instructions to perform some or all of the operations of the controller 220.

The program may be installed in the portable terminal 4000 in advance, or the user of the portable terminal 4000 may install the program by downloading the program from a server that provides an application. A recording medium that stores the program may be included in the server that provides the application.

In the meantime, the X-ray detector 130 may be implemented as a fixed type of X-ray detector 130-1 fixed on a stand 20 or a table 12, or may detachably equipped in the installation part 14 or 24, or alternatively, the X-ray detector 200 may be implemented as a mobile X-ray detector 130-2 or a portable X-ray detector available at any place. The mobile X-ray detector 130-2 or the portable X-ray detector may be implemented in a wired type or a wireless type depending on the data transmission method and the power supplying method.

The X-ray detector 130 may or may not be included as an element of the X-ray system 1000. In the latter case, the X-ray detector 130 may be registered in the X-ray system 1000 by the user. Furthermore, in both cases, the X-ray detector 130 may be connected to the controller 220 through the communication interface 210 to receive a control signal or transmit image data.

The user input interface 160 may be arranged on one side of the X-ray irradiator 120 to provide information for the user and receive a command from the user. The user input interface 160 may be a sub user interface that performs part or all of the functions performed by the input interface 240 and the output interface 250 of the workstation 200.

In a case that all or some of the components of the communication interface 210 and the controller 220 are arranged separately from the workstation 200, they may be included in the user input interface 160 arranged in the X-ray irradiator 120.

The X-ray system 1000 illustrated in FIG. 1 is a room X-ray imaging device connected to the ceiling of the examination room, but the X-ray system 1000 may include variously structured X-ray devices such as a C-arm type X-ray device, a mobile X-ray device, etc., within a range that is obvious to those of ordinary skill in the art.

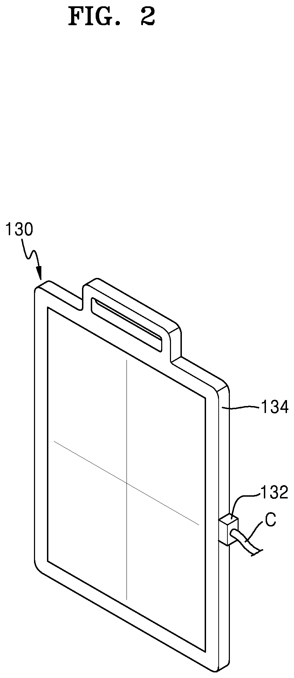

FIG. 2 is an exterior view of the X-ray detector 130.

Referring to FIG. 2, the X-ray detector 130 may be implemented as a mobile X-ray detector. In this case, the X-ray detector 130 may include a battery that supplies power and operate wirelessly, or as illustrated in FIG. 2, may have a charging port 132 connected to a separate power supply via a cable C and operate.

Within a case 304 that defines an exterior of the X-ray detector 130, a detection element that detects an X-ray and converts it to image data, a memory that temporarily or non-temporarily stores the image data, a communication module that receives a control signal from the X-ray system 1000 or transmits the image data to the X-ray system 1000, and a battery. Furthermore, the memory may store image correction information of the detector and unique identification information of the X-ray detector 130, and transmit the stored identification information while communicating with the X-ray system 1000.

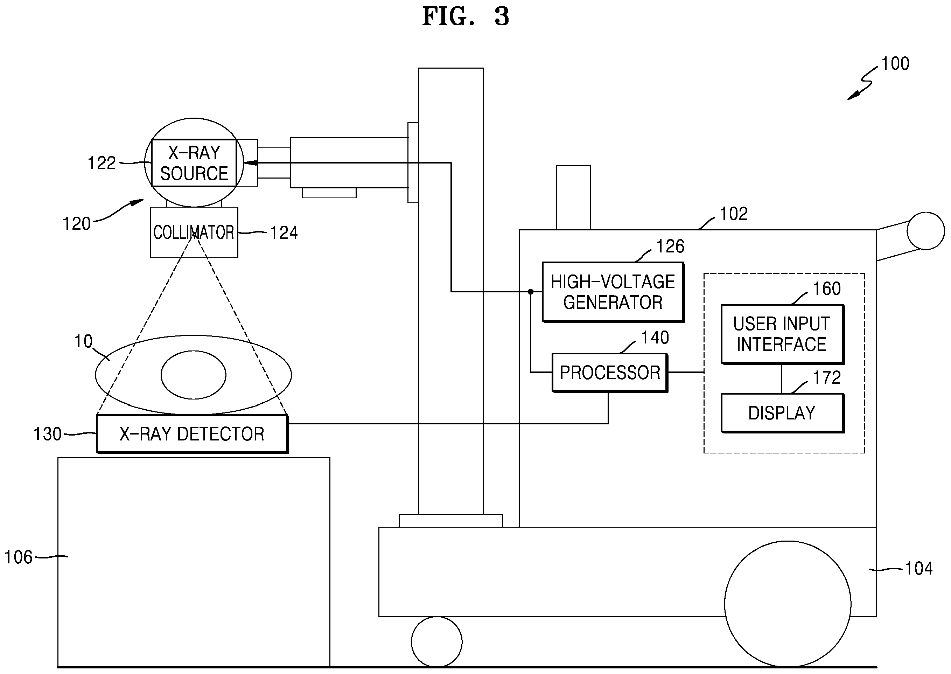

FIG. 3 illustrates an X-ray imaging device 100 including the mobile X-ray detector 130, according to an embodiment of the present disclosure.

Referring to FIG. 3, the X-ray imaging device 100 may include the mobile X-ray detector 130. The mobile X-ray detector 130 is a mobile or portable type of X-ray detector that may perform X-raying without being restricted by an imaging location. The X-ray imaging device 100 illustrated in FIG. 3 may be an embodiment of the X-ray imaging device 100 as illustrated in FIG. 1. Among the components included in the X-ray imaging device 100 illustrated in FIG. 3, the same components as in FIG. 1 use the same reference numerals and the redundant description will not be repeated.

The X-ray imaging device 100 illustrated in FIG. 3 may include a main unit 102 that includes a processor 140 for controlling general operation of the X-ray imaging device 100, a moving unit 104 with wheels arranged to move the X-ray imaging device 100, a table 106, the X-ray irradiator 120 for generating and irradiating X-rays to an object, the X-ray detector 130 for detecting X-rays irradiated by the X-ray irradiator 120 to the object and transmitted through the object, a user input interface 160 for receiving a user input, and a display 172.

The main unit 102 may further include an operation unit for providing a user interface to operate the X-ray imaging device 100. Although the operation unit is illustrated in FIG. 3 as being included in the main unit 102, it is not limited thereto. For example, as illustrated in FIG. 1, the input interface 240 and the output interface 250 of the X-ray system 1000 may be arranged on one side of the workstation 200 (see FIG. 1).

The X-ray irradiator 120 may include an X-ray source 122 for generating X-rays, and a collimator 124 for controlling an irradiation area of the X-rays generated and irradiated by the X-ray source 122 by guiding the path of the X-rays. The main unit 102 may include a high-voltage generator 126 for generating a high voltage to be applied to the X-ray source 122.

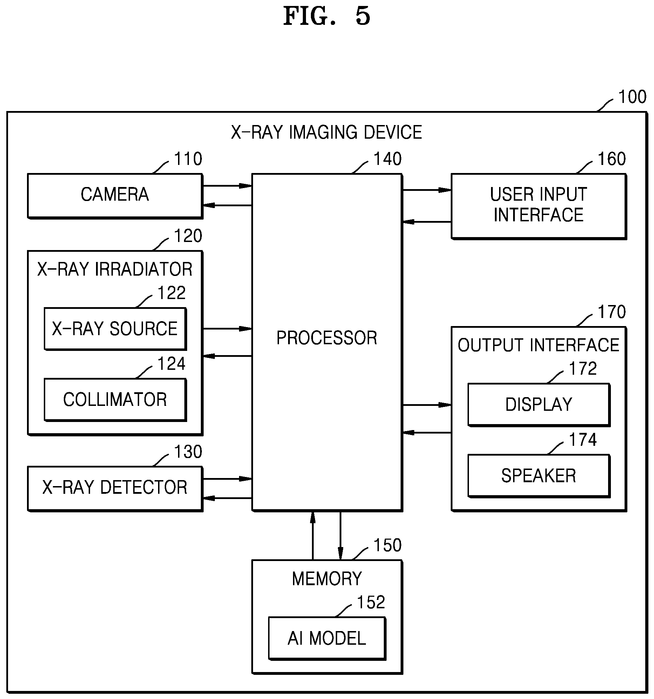

Specific functions and/or operations of the X-ray detector 130, the processor 140, the user input interface 160 and the display 172 will be described in detail in connection with FIG. 5.

It is also possible that the X-ray system 1000 is implemented not only in the aforementioned ceiling type but also in a mobile type. The X-ray detector 130 of FIG. 3 is illustrated as a table type that is placed on the table 106, but it is obvious that it may also be implemented in a stand type as a mobile type or a portable type.

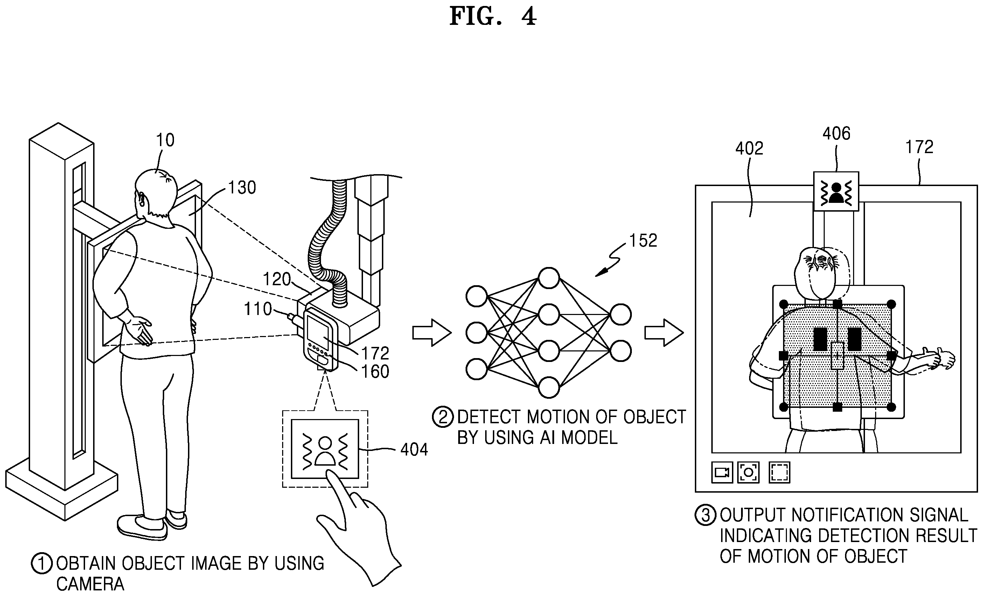

FIG. 4 is a conceptual diagram for describing an operation of the X-ray imaging device 100 for detecting a motion of the object 10 from an image obtained through the camera 110, according to an embodiment of the present disclosure.

In FIG. 4, the X-ray imaging device 100 is illustrated as a ceiling type, but is not limited thereto. In an embodiment of the present disclosure, the X-ray imaging device 100 may be implemented in a mobile type.

Referring to FIG. 4, the X-ray imaging device 100 may include the camera 110, the X-ray irradiator 120, the X-ray detector 130, the user input interface 160, and the display 172. In FIG. 4, only minimal components for describing the function and/or operation of the X-ray imaging device 100 are illustrated, and components included in the X-ray imaging device 100 are not limited to those illustrated in FIG. 4. The components of the X-ray imaging device 100 will be described in detail in connection with FIG. 5.

In operation ①, the X-ray imaging device 100 obtains an object image 402 by photographing the object 10 with the camera 110. The X-ray imaging device 100 may capture an image of the object 10 through the camera 110 as patient positioning in front of the X-ray detector 130 is completed. In an embodiment of the present disclosure, the X-ray imaging device 100 may display a button UI 404 for performing a motion detection mode on the display 172 and receive a touch input of the user touching the button UI 404. In response to the user's touch input of the user being received, the X-ray imaging device 100 may perform the motion detection mode, and obtain the object image 402 by photographing the object 10 through the camera 110. It is not, however, limited thereto, and the X-ray imaging device 100 may automatically perform the motion detection mode. In an embodiment of the present disclosure, the X-ray imaging device 100 may automatically perform the motion detection mode after a lapse of a preset time after the patient positioning in front of the X-ray detector 130 is completed, and obtain the object image by photographing the object 10 through the camera 110.

The obtained object image 402 is a two-dimensional (2D) image obtained through the camera 110 having a general image sensor (e.g., CMOS or CCD), which is different from an X-ray image obtained by receiving X-rays transmitted through the object 10 through the X-ray detector 130 and performing image processing on it. In an embodiment of the present disclosure, the X-ray imaging device 100 may display the object image 402 on the display 172.

The X-ray imaging device 100 detects a motion of the object by using an Al model 152, in operation ②. The X-ray imaging device 100 may detect a motion of the object by analyzing the obtained object image 402 with the use of the Al model 152. In an embodiment of the present disclosure, the X-ray imaging device 100 may determine a first image frame obtained by photographing the object 10 after the patient positioning is completed as a reference image, and detect a motion of the object by using the Al model 152 to compare an image frame obtained through subsequent image photographing with the reference image. The Al model 152 may include at least one of a machine learning algorithm and a deep neural network model.

In an embodiment of the present disclosure, the X-ray imaging device 100 may use a self-organizing map of the machine learning model to cluster pixels of the object 10 and background in the reference image and the subsequent image frame and apply weights to pixels that represent the object 10, thereby increasing accuracy in motion detection of the object 10 in a way of reducing the influence of background noise.

In an embodiment of the present disclosure, the X-ray imaging device 100 may detect a motion of the object by inputting the object image 402 to a trained deep neural network model and performing inferencing using the deep neural network model. The X-ray imaging device 100 may extract key points of a landmark of the object 10 from each of the reference image and the subsequent image frame by performing inferencing using the deep neural network model. The deep neural network model may be a model trained by a supervised learning method that applies a plurality of obtained images as input data and applies location coordinates of key points of the landmark as ground truth. The deep neural network model may be, for example, a convolutional neural network (CNN) model, but is not limited thereto. The X-ray imaging device 100 may calculate a difference between key points extracted from the reference image and key points extracted from the subsequent image frame, and detect a motion of the object by comparing the calculated difference with a threshold.

The X-ray imaging device 100 outputs a notification signal that indicates a result of the detecting of the motion of the object 10, in operation ③. In an embodiment of the present disclosure, the X-ray imaging device 100 may display a graphical user interface (UI) 406 having a preset color to indicate the motion of the object 10 on the display 172. The graphical UI 406 may be an icon that has, for example, orange (or red) color and shapes a moving person. Although not illustrated in FIG. 4, the X-ray imaging device 100 may further include a speaker 174 configured to output an acoustic signal, and output at least one acoustic signal among a voice and a notification sound that notifies the user of information about the motion of the object 10 through the speaker 174.

The conventional X-ray imaging device detects a motion of the object 10 (e.g., a patient) by attaching a fiducial element onto a certain body portion of the object 10 and analyzing a displacement of the fiducial element from an image obtained by photographing the object 10 with the fiducial element attached thereto. The conventional art does not work when there is no fiducial element to be attached to a certain body portion of the object, and may detect a motion of the object 10 only in a procedure for obtaining successive X-ray images. The conventional art has a limitation in that it is unable to prevent an abnormal image from being obtained by detecting a motion of the object 10 before taking an X-ray image.

The present disclosure aims to provide the X-ray imaging device 100 and an operation method thereof, which detects a motion of the object 10 by obtaining the object image 402 by photographing the object 10 with the camera 110 and analyzing the object image 402 by using the Al model 152.

In an embodiment illustrated in FIG. 4, unlike the conventional art, by outputting the graphical UI 406 or an acoustic signal that indicates a motion of the object 10 (e.g., a patient) in a case that the motion is bigger than a normal motion of the object 10 is made such as breathing before actual X-raying is taken after patient positioning is completed, the X-ray imaging device 100 may assist the user to take efficient and precise X-raying and thus, increase user convenience. In an embodiment of the present disclosure, the X-ray imaging device 100 may automate patient monitoring, and obtain an X-ray image of the object 10 while the object 10 remains in as precise a position as the user (e.g., a radiographer) intends, thereby preventing deterioration of image quality of the X-ray image due to a motion of the object 10. Furthermore, in an embodiment of the present disclosure, the X-ray imaging device 100 provides a technical effect of preventing an increase in radiography time and a risk of extra radiation exposure that occurs in retaking caused by a motion of the object 10.

FIG. 5 is a block diagram illustrating components of the X-ray imaging device 100, according to an embodiment of the present disclosure.

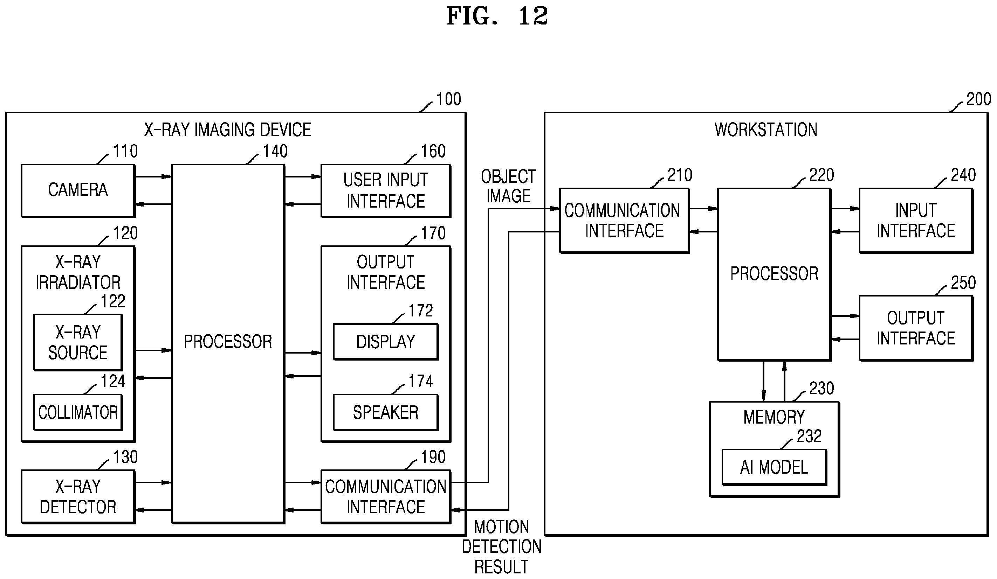

The X-ray imaging device 100 illustrated in FIG. 5 may be a mobile-type device including the mobile X-ray detector 130. It is not, however, limited thereto, and the X-ray imaging device 100 may be implemented in a ceiling type. The X-ray imaging device 100 of the ceiling type will be described in detail later in connection with FIG. 12.

Referring to FIG. 5, the X-ray imaging device 100 may include the camera 110, the X-ray irradiator 120, the X-ray detector 130, the processor 140, the memory 150, the user input interface 160, and the output interface 170. The camera 110, the X-ray irradiator 120, the X-ray detector 130, the processor 140, the memory 150, the user input interface 160, and the output interface 170 may be electrically and/or physically connected to one another. In FIG. 5, only essential components for describing an operation of the X-ray imaging device 100 are shown, and components included in the X-ray imaging device 100 are not limited to those illustrated in FIG. 5. In an embodiment of the present disclosure, the X-ray imaging device 100 may further include a communication interface 190 (see FIG. 12) for performing data communication with the workstation 200 (see FIG. 12), the server 2000 (see FIG. 1), the other medical device 3000 (see FIG. 1) or the external portable terminal 4000 (see FIG. 1). In an embodiment of the present disclosure, the X-ray imaging device 100 may further include the high-voltage generator 126 (see FIG. 3) for generating a high voltage to be applied to the X-ray source 122. In another embodiment of the present disclosure, the output interface 170 of the X-ray imaging device 100 may not include the speaker 174.

The camera 110 is configured to obtain an object image by photographing the object (e.g., a patient) positioned in front of the X-ray detector 130. In an embodiment of the present disclosure, the camera 110 may include a lens module, an image sensor and an image processing module. The camera 110 may obtain a still image or a video about the object through the image sensor (e.g., CMOS or CCD). The video may include a plurality of image frames obtained in real time by shooting the object through the camera 110. The image processing module may encode a still image having a single image frame or video data comprised of a plurality of image frames obtained through the image sensor and send it to the processor 140.

In an embodiment of the present disclosure, the camera 110 may be implemented as a form factor to be mounted on one side of the user input interface 160 of the X-ray imaging device 100, and may be a light-weighted RGB camera that consumes low power. It is not, however, limited thereto, and in another embodiment of the present disclosure, the camera 110 may be implemented as any type of camera such as an RGB-depth camera including a depth estimation function, a stereo fish-eye camera, a gray-scale camera or an infrared camera.

The X-ray irradiator 120 is configured to generate X-rays and irradiate the X-rays onto an object. The X-ray irradiator 120 may include the X-ray source 122 that generates X-rays by receiving a high voltage generated from the high-voltage generator 126 (see FIG. 3) and irradiates the X-rays, and the collimator 124 that adjusts an X-ray irradiation area by guiding the path of the X-rays irradiated from the X-ray source 122.

The X-ray source 122 may include an X-ray tube, and the X-ray tube may be implemented as a two-pole vacuum tube with anode and cathode. The inside of the X-ray tube is made into a high vacuum state of about 10 mmHg, and thermoelectrons are generated by heating a cathode filament. For the filament, a tungsten filament may be used, and the filament may be heated by applying a voltage of 10 V and a current of about 3 to 5 A to an electric wire connected to the filament. When a high voltage of about 10 to 300 kVp is applied between the cathode and the anode, the thermoelectrons are accelerated and collide with a target material at the anode, producing X-rays. The X-rays may be irradiated to the outside through a window, and a barium thin film may be used as a material of the window. In this case, most of energy of the electrons colliding with the target material is consumed as heat, and a remnant of the energy is converted to the X-rays.

The anode is mainly comprised of copper, and the target material is arranged on a side opposite the cathode, and for the target material, high resistant materials such as Cr, Fe, Co, Ni, W, Mo, etc., may be used. The target material may be rotated by a rotating magnetic field, and when the target material is rotated, an electron impact area increases and a heat accumulation rate may increase 10 times or more per unit area as compared to an occasion when the target material is fixed.

The voltage applied between the cathode and the anode of the X-ray tube is called a tube voltage, which is applied from the high-voltage generator 126 and the magnitude may be expressed as a crest value kVp. When the tube voltage increases, the velocity of the thermoelectrons increases, and as a result, energy of the X-rays generated from colliding with the target material (photon energy) increases. The current flowing in the X-ray tube is called a tube current, which may be expressed in average mA, and with an increase in tube current, the number of thermoelectrons emitted from the filament increases and as a result, the dose of X-rays (number of X-ray photons) generated from colliding with the target material increases. Accordingly, X-ray energy may be controlled by the tube voltage, and the intensity or dose of the X-rays may be controlled by the tube current and the X-ray exposure time.

The X-ray detector 130 is configured to detect X-rays irradiated by the X-ray irradiator 120 and transmitted through the object. In an embodiment of the present disclosure, the X-ray detector 130 may be a digital detector implemented with a charge coupled device (CCE) or implemented with a thin film transistor (TFT). Although the X-ray detector 130 is illustrated in FIG. 5 as a component included in the X-ray imaging device 100, the X-ray detector 130 may be a separate device that is attachable to and detachable from the X-ray imaging device 100.

The processor 140 may execute one or more instructions of a program stored in the memory 150. The processor 140 may include hardware components for performing arithmetic, logical, and input/output operations and image processing. The processor 140 is illustrated as one element in FIG. 5, but is not limited thereto. In an embodiment of the present disclosure, the processor 140 may be configured with one or more elements. The processor 140 may be a universal processor such as a central processing unit (CPU), an application processor (AP), a digital signal processor (DSP), etc., a dedicated graphic processor such as a graphic processing unit (GPU), a vision processing unit (VPU), etc., or a dedicated artificial intelligence (AI) processor such as a neural processing unit (NPU). The processor 140 may control processing of input data according to a predefined operation rule or an Al model. When the processor 140 is the dedicated Al processor, the dedicated Al processor may be designed in a hardware structure specialized for processing with a particular Al model.

The memory 150 may include, for example, at least one type of storage media including a flash memory, a hard disk, a multimedia card micro type memory, a card type memory (e.g., SD or XD memory), a random access memory (RAM), a static random access memory (SRAM), a read-only memory (ROM), an electrically erasable programmable read-only memory (EEPROM), a programmable read-only memory (PROM), or an optical disk.

Instructions related to functions and/or operations of the X-ray imaging device 100 for detecting a motion of the object from the object image obtained by the camera 110 may be stored in the memory 150. In an embodiment of the present disclosure, the memory 150 may store at least one of algorithms, data structures, program codes, application programs, and instructions that are readable to the processor 140. The instructions, algorithms, data structures and program codes stored in the memory 150 may be implemented in e.g., a programming or scripting language such as C, C++, Java, assembler, etc.

In the following embodiments, the processor 140 may be implemented by executing the instructions or program codes stored in the memory 150.

The processor 140 may obtain image data of the object image obtained by photographing the object from the camera 110. The processor 140 may obtain the image data of the object by controlling the camera 110 to photographing the object as patient positioning in front of the X-ray detector 130 is completed. In an embodiment of the present disclosure, the processor 140 may control the camera 110 to photograph the object through the camera 110 in response to a user input for performing the motion detection mode being received through the user input interface 160. The user input interface 160 may receive a user touch input that selects the button UI 404 (see FIG. 4) for performing the motion detection mode, which is displayed on the display 172, and on receiving the touch input, the processor 140 may perform the motion detection mode (manual mode). In an embodiment of the present disclosure, the user input to perform the motion detection mode is not limited to the touch input, but may correspond to an input that presses a key pad, a hardware button, a jog switch, etc.

It is not, however, limited thereto, and the processor 140 may automatically perform the motion detection mode to photograph the object. In an embodiment of the present disclosure, the processor 140 may automatically perform the motion detection mode after a lapse of preset time after the patient positioning in front of the X-ray detector 130 is completed (automatic mode).

In an embodiment of the present disclosure, the processor 140 may obtain video data comprised of a plurality of image frames obtained by the camera 110 in real time.

The processor 140 may detect a motion of the object from image data by analyzing the image data using the artificial intelligence (AI) model 152. The Al model 152 may include at least one of a machine learning algorithm and a deep neural network. In an embodiment of the present disclosure, the Al model 152 may be implemented with the instructions, program codes or algorithms stored in the memory 150, but is not limited thereto. In an embodiment of the present disclosure, the Al model 152 may not be included in the X-ray imaging device 100. In this case, an Al model 232 (see FIG. 12) may be included in the workstation 200 (see FIG. 12).

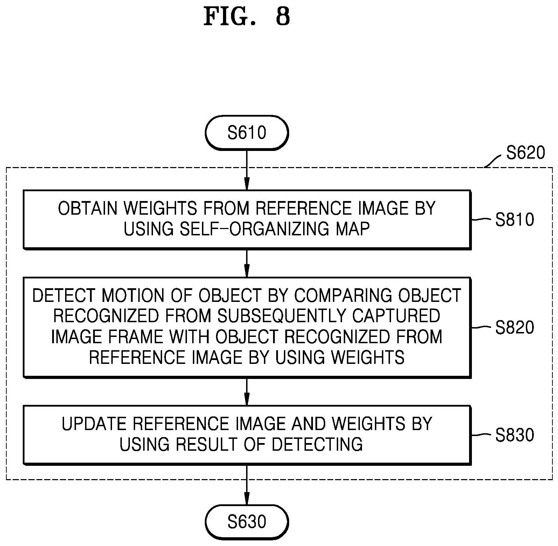

As the motion detection mode is performed after the patient positioning is completed, the processor 140 may determine a first image frame obtained by photographing the object using the camera 110 as a reference image, and detect a motion of the object by comparing, using the Al model 152, an image frame obtained through subsequent image photographing with the reference image. In an embodiment of the present disclosure, the processor 140 may use a self-organizing map, which is a machine learning algorithm of the Al model 152, to cluster pixels of the object and the background, respectively, from each of the reference image and the subsequent image frame, and apply weights to pixels that represent the object, thereby detecting a motion of the object. By using the self-organizing map, the influence of background noise may be reduced, so that the motion detection accuracy of the object may be improved. A specific embodiment in which the processor 140 detects a motion of the object by using the self-organizing map will be described in FIG. 8 in detail.

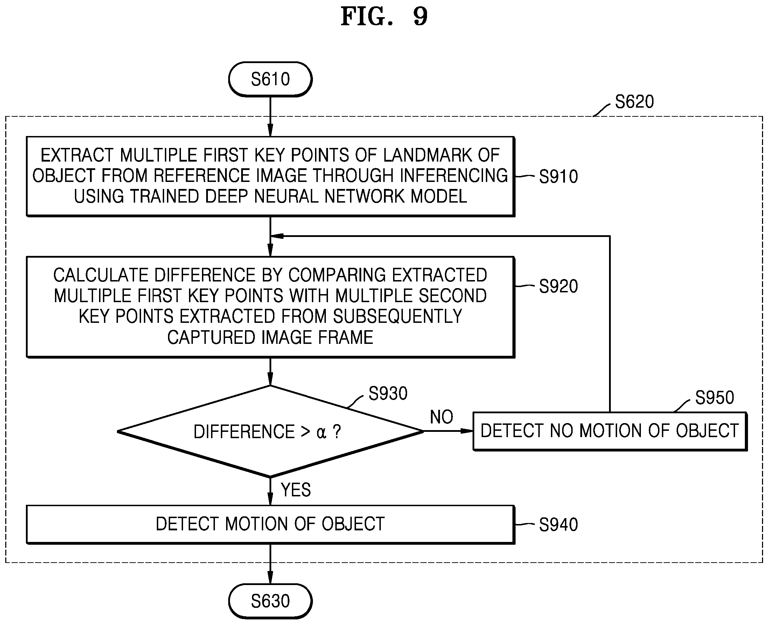

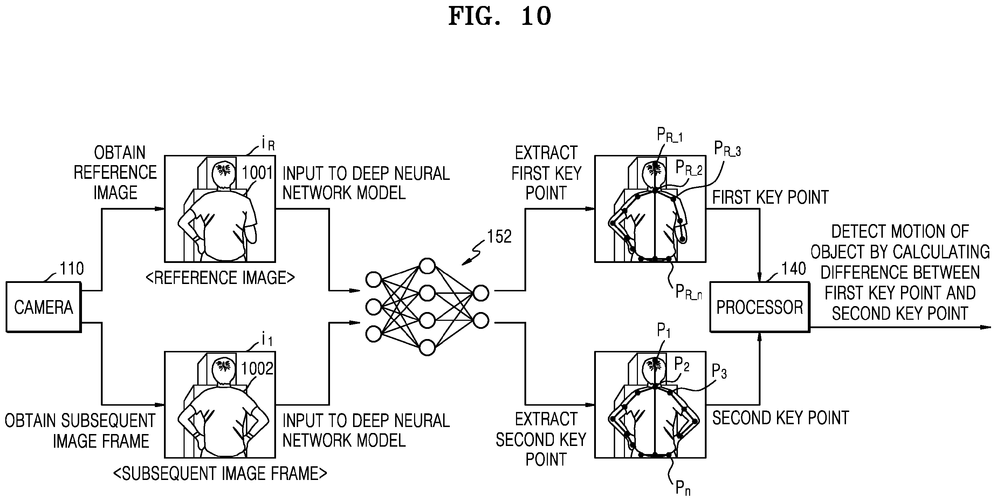

In an embodiment of the present disclosure, the processor 140 may detect a motion of the object by inputting the object image to a trained deep neural network model of the Al model 152, and performing inferencing using the deep neural network model. The processor 140 may extract key points of a landmark of the object from each of the reference image and the subsequent image frame by performing inferencing using the deep neural network model. The deep neural network model may be a model trained by a supervised learning method that applies a plurality of obtained images as input data and applies location coordinates of key points of the landmark as ground truth. The deep neural network model may be, for example, a convolutional neural network (CNN) model, but is not limited thereto. The processor 140 may calculate a difference between key points extracted from the reference image and key points extracted from the subsequent image frame, and detect a motion of the object by comparing the calculated difference with a threshold. A specific embodiment in which the processor 140 detects a motion of the object by using the deep neural network model will be described in connection with FIGS. 9 and 10 in detail.

The processor 140 may control the output interface 170 to output a notification signal that notifies the user of a detection result of a motion of the object. In an embodiment of the present disclosure, the processor 140 may control the display 172 to display a graphical UI having a preset color that represents a motion of the object. The graphical UI may be an icon that has, for example, orange (or red) color and shapes a moving person. In an embodiment of the present disclosure, the processor 140 may control the speaker 174 to output at least one acoustic signal among a voice and a notification sound that notifies the user of information about the motion of the object.

The processor 140 may set a motion detection sensitivity for adjusting the level of motion detection. The motion detection sensitivity indicates a degree about whether to provide a motion detection and notification signal depending on the degree of motion of the object. A motion is detected and a notification signal is output even with a small motion of the object the larger the motion detection sensitivity, and a motion is detected only when the object makes a relatively big motion the smaller the motion detection sensitivity. In an embodiment of the present disclosure, the processor 140 may set the motion detection sensitivity based on at least one of a source to image distance (SID), which is a distance between the object and the X-ray irradiator 120, the size and shape of the object, and an imaging protocol. It is not, however, limited thereto, and the processor 140 may set the motion detection sensitivity according to a user input. In this case, the user input interface 160 may receive a user input to set or adjust the motion detection sensitivity, and the processor 140 may set the motion detection sensitivity based on the received user input.

The user input interface 160 is configured to provide an interface for operating the X-ray imaging device 100. The user input interface 160 may be configured as, for example, but not exclusively, a control panel including hardware elements such as a keypad, a mouse, a track ball, a jog dial, a jog switch or a touch pad. In an embodiment of the present disclosure, the user input interface 160 may be configured as a touch screen that receives a touch input and displays a graphical user interface (GUI).

The user input interface 160 may receive an input of a command to operate the X-ray imaging device 100 and various information about X-raying from the user. The user input interface 160 may receive a user input such as a command to for example, set the motion detection sensitivity, perform the motion detection mode (manual mode), etc.

The output interface 170 is configured to output a detection result of a motion of the object under the control of the processor 140. The output interface 170 may include a display 172 and a speaker 174.

The display 172 may display the GUI that represents the detection result of the motion of the object. The display 172 may include a hardware device including at least one of e.g., a CRT display, an LCD display, a PDP display, an OLED display, an FED display, an LED display, a VFD display, a DLP display, a flat panel display, a 3D display, and a transparent display, but is not limited thereto. In an embodiment of the present disclosure, the display 172 may be configured as a touch screen including a touch interface. In a case that the display 172 is configured with a touch screen, the display 172 may be a component integrated with the user input interface 160 comprised of a touch panel.

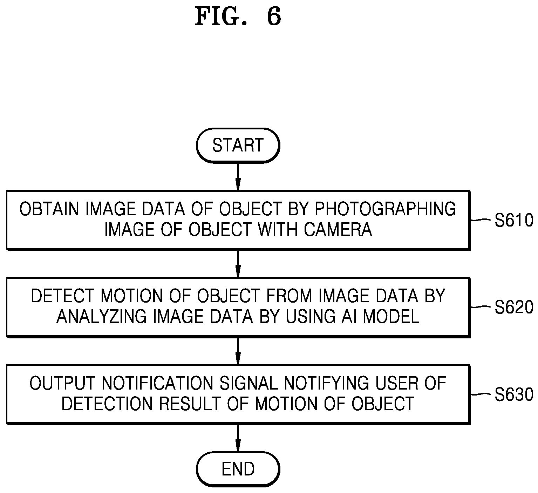

FIG. 6 is a flowchart illustrating a method by which the X-ray imaging device 100 detects a motion of an object from an image obtained through a camera, according to an embodiment of the present disclosure.

In operation S610, the X-ray imaging device 100 obtains image data of the object by photographing the object using a camera. The X-ray imaging device 100 may obtain image data by photographing the object (e.g., a patient) positioned in front of the X-ray detector 130 (see FIG. 4), using the camera. In an embodiment of the present disclosure, the X-ray imaging device 100 may receive a user input to select the button UI for performing the motion detection mode after the patient positioning is completed, and perform the motion detection mode based on the received user input. The X-ray imaging device 100 may obtain image data of the object through the camera 110 to detect a motion of the object as the motion detection mode is performed.

In an embodiment of the present disclosure, the X-ray imaging device 100 may automatically perform the motion detection mode after a lapse of a preset time after the patient positioning in front of the X-ray detector 130 is completed. The X-ray imaging device 100 may obtain image data by photographing the object through the camera 110 as the motion detection mode is performed.

The X-ray imaging device 100 may obtain a reference image by photographing the object after the patient positioning in front of the X-ray detector 130 is completed, and obtain an image frame by taking a subsequent image of the object after the reference image is obtained. In an embodiment of the present disclosure, the X-ray imaging device 100 may obtain a plurality of image frames by using the camera to take images of the object in real time after obtaining the reference image.

In operation S620, the X-ray imaging device 100 may detect a motion of the object from image data by analyzing the image data using an Al model. The X-ray imaging device 100 may detect a motion of the object by comparing the object recognized from the reference image with the object recognized from the subsequently captured image through the Al model based analysis.

In an embodiment of the present disclosure, the X-ray imaging device 100 may recognize an object from each of the reference image and the subsequent image frame by using a self-organizing map, which is a machine learning algorithm of the Al model, cluster pixels that represent the recognized object and the background, respectively, and detect a motion of the object by applying weights to pixels that represent the object.

In an embodiment of the present disclosure, the X-ray imaging device 100 may input the image data to a trained deep neural network model among Al models, and detect a motion of the object by performing inferencing using the deep neural network model. The X-ray imaging device 100 may extract key points of a landmark of the object from each of the reference image and the subsequent image frame by performing inferencing using the deep neural network model. The X-ray imaging device 100 may calculate a difference between key points extracted from the reference image and key points extracted from the subsequent image frame, and detect a motion of the object by comparing the calculated difference with a threshold.

In operation S630, the X-ray imaging device 100 outputs a notification signal to notify the user of a detection result of a motion of the object. In an embodiment of the present disclosure, the X-ray imaging device 100 may display a graphical UI having a preset color that represents the motion of the object. In an embodiment of the present disclosure, the X-ray imaging device 100 may output at least one acoustic signal among a voice and a notification sound that notifies the user of information about the motion of the object.

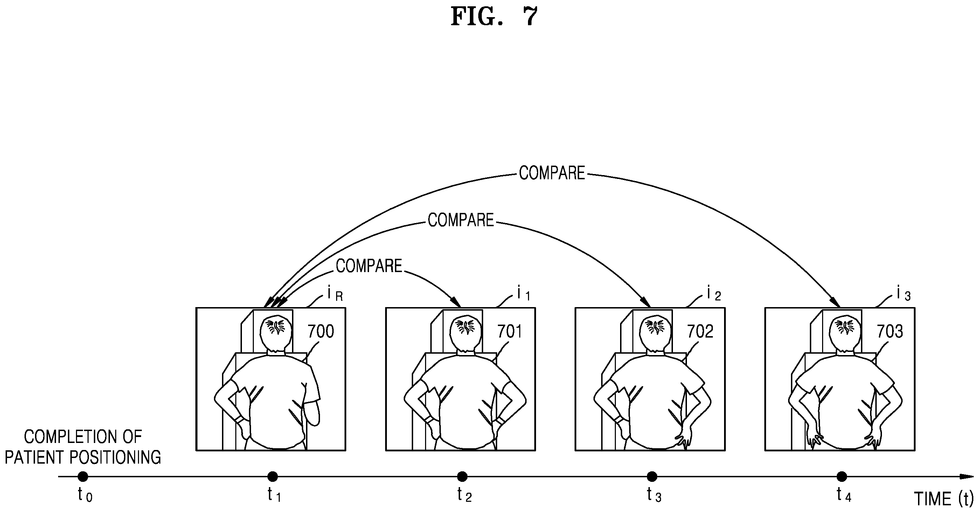

FIG. 7 is a diagram for describing an operation of the X-ray imaging device 100 for detecting a motion of an object by comparing a reference image i_{R} with subsequent image frames i₁, i₂, i₃, ..., according to an embodiment of the present disclosure.

Referring to FIG. 7, patient positioning that an object is positioned in front of the X-ray detector 130 (see FIGS. 4 and 5) is completed at time t₀. The X-ray imaging device 100 may obtain a plurality of image frames i_{R}, i₁, i₂, i₃, ... by photographing the object after the patient positioning is completed, using the camera. The X-ray imaging device 100 may determine an image frame obtained at first time t₁ after the patient positioning as the reference image i_{R}. The X-ray imaging device 100 may store the reference image i_{R} in a storage space in the memory 150 (see FIG. 5). The X-ray imaging device 100 may obtain the plurality of image frames i₁, i₂, i₃, ... by taking subsequent images of the object after obtaining the reference image i_{R}. For example, the X-ray imaging device 100 may obtain the first image frame i₁ at the second time t₂, obtain the second image frame i₂ at the third time t₃, and obtain the third image frame i₃ at the fourth time t₄.

The X-ray imaging apparatus 100 may recognize an object 700 from the reference image i_{R} through the Al model 152 (see FIGS. 4 and 5) based analysis, and detect a motion of the object by comparing objects 701, 702 and 703 recognized from the plurality of image frames i₁, i₂, i₃, ... obtained through subsequent image taking. For example, the X-ray imaging device 100 may recognize, by using the Al model 152, the object 700 from the reference image i_{R}, recognize the object 701 from the first image frame i₁ that is captured subsequently, and detect a motion of the object by comparing the objects recognized from the reference image i_{R} and the first image frame i₁, respectively. Similarly, the X-ray imaging device 100 may detect a motion of the object by comparing the object 700 recognized from the reference image i_{R} with each of the object 702 recognized from the second image frame i₂ and the object 703 recognized from the third image frame i₃.

FIG. 8 is a flowchart illustrating a method by which the X-ray imaging device 100 detects a motion of an object by using a machine learning algorithm, according to an embodiment of the present disclosure.

Operations S810 to S830 illustrated in FIG. 8 are detailed operations of operation S620 illustrated in FIG. 6. After operation S610 illustrated in FIG. 6 is performed, operation S810 of FIG. 8 may be performed. Operation S830 of FIG. 8 may be followed by operation S630 illustrated in FIG. 6.

In operation S810, the X-ray imaging device 100 obtains weights from the reference image by using a self-organizing map. The processor 140 (see FIG. 5) of the X-ray imaging device 100 may recognize an object from the reference image by using the self-organizing map among machine learning algorithms, and apply weights to pixels that represent the recognized object. In an embodiment of the present disclosure, the processor 140 may store the image data and weights of the reference image in a storage space in the memory 150 (see FIG. 5). In an embodiment of the present disclosure, the processor 140 may not apply any weight or may apply low weights to pixels that represent the background.

In operation S820, using the weights, the X-ray imaging device 100 detects a motion of the object by comparing the object recognized from the subsequently captured image frame with the object recognized from the reference image. In an embodiment of the present disclosure, the processor 140 of the X-ray imaging device 100 may recognize an object from an image frame obtained by subsequent image taking after obtaining the reference image, and calculate a difference in image pixel value between the recognized objects by comparing the recognized object with the object recognized from the reference image. The processor 140 may recognize a motion of the object when the calculated difference exceeds a preset threshold. In an embodiment of the present disclosure, the processor 140 may periodically recognize an object from the subsequent image frame at preset time intervals, and detect a motion of the object by comparing the recognized object with the object recognized from the reference image.

In operation S830, the X-ray imaging device 100 update the reference image and the weights by using a result of the detection. The processor 140 of the X-ray imaging device 100 may update the reference image by using information about pixels of the respective objects recognized from the reference image and each of the subsequently captured image frames. Furthermore, the processor 140 may update the weights by using the information about the pixels of the detected object. The processors 140 may update the reference image and weights by repeatedly performing operations S820 and S830 for a plurality of subsequently captured image frames.

In a case of recognizing an object based on the self-organizing map, high weights may be applied to the object in the image while no or relatively low weights are applied to the background. According to the flowchart as illustrated in FIG. 8, by using the self-organizing map to recognize an object and updating the reference image and weights, the X-ray imaging device 100 may minimize the influence of random background noise from external conditions such as a low illuminance environment and a motion detection level depending on the body type of the patient or a difference in imaging distance, thereby increasing the accuracy in detection of the motion of the object.

In an embodiment of the present disclosure, the X-ray imaging device 100 may detect a motion of the object by comparing, using a well-known machine learning algorithm, the reference image with a subsequent image frame. For example, the X-ray imaging device 100 may detect a motion of the object by using at least one of support vector machine (SVM), linear regression, logistic regression, Naive Bayes, random forest, decision tree or k-nearest neighbor algorithm to analyze the reference image and the subsequent image frame.

FIG. 9 is a flowchart illustrating a method by which the X-ray imaging device 100 detects a motion of an object by using a trained deep neural network, according to an embodiment of the present disclosure.

Operations S910 to S95 illustrated in FIG. 9 are detailed operations of operation S620 illustrated in FIG. 6. After operation S610 illustrated in FIG. 6 is performed, operation S910 of FIG. 9 may be performed. Operation S940 of FIG. 9 may be followed by operation S630 illustrated in FIG. 6.

FIG. 10 is a conceptual diagram for describing an operation of the X-ray imaging device 100 for detecting a motion of an object by using a trained deep neural network model, according to an embodiment of the present disclosure.

A function and/or operation of the X-ray imaging device 100 for detecting a motion of an object will now be described with reference to both FIGS. 9 and 10.

In operation S910, the X-ray imaging device 100 extracts a plurality of first key points of a landmark of the object from the reference image through inferencing using a trained deep neural network model Also referring to FIG. 10, the X-ray imaging device 100 may obtain the reference image i_{R} through the camera 110. The processor 140 of the X-ray imaging device 100 may input the reference image i_{R} to the Al model 152, and may extract a plurality of first key points P_{R_1}, P_{R_2}, ..., P_{R_n} of a landmark of an object 1001 from the reference image i_{R} through inferencing using the Al model 152. in an embodiment of the present disclosure, the locations and number of landmarks of the object may be determined according to an imaging protocol. For example, in a case of a stitching protocol to capture images of the whole body of an object (e.g., a patient), among the body portions, head, shoulders, elbows, hands, waist, knees and feet may be determined as the landmarks, and the processor 140 may extract key points of the landmarks. For example, in a case of a whole spine protocol, among the body portions from ears to below the pelvis, head, shoulders, elbows, hands, waist, etc., may be determined as the landmarks, and the processor 140 may extract key points of the landmarks. In a case of an extremity protocol for head (skull), hands or feet, portions with unique body characteristics such as the face, hands, or feet may be determined as the landmarks, and the processor 140 may extract key points of the landmarks. FIG. 10 illustrates the plurality of first key points P_{R_1}, P_{R_2}, ... , P_{R_n} extracted according to the whole spine protocol, for convenience of explanation. The plurality of first key points P_{R_1}, P_{R_2}, ... , P_{R_n} are not limited to those illustrated in FIG. 10, and may be changed depending on the imaging protocol and required accuracy. In an embodiment of the present disclosure, the processor 140 may build the plurality of first key points P_{R_1}, P_{R_2}, ... , P_{R_n} as a dataset. The processor 140 may store the dataset in the memory 150 (see FIG. 5).

In an embodiment of the present disclosure, the Al model 152 may be a deep neural network model. The deep neural network model may be a model trained by a supervised learning method that applies a plurality of obtained images as input data and applies location coordinates of key points of the landmarks as ground truth. To increase accuracy in extraction of the key points, the deep neural network model may be trained in a partially modified form. When the input data (e.g., a plurality of images) and ground truth data (e.g., location coordinates of key points) for training are insufficient, it is also possible to process the data by augmentation to increase an amount of the data or apply a fine tuning method that partially modifies the trained model.

The deep neural network model may be implemented as a convolutional neural network (CNN) model. The deep neural network model may be, for example, U-Net. It is not, however, limited thereto, and the deep neural network model may be implemented as any pose estimation model that is known to the public.

Referring back to FIG. 9, in operation S920, the X-ray imaging device 100 calculates a difference by comparing the plurality of first key points extracted from the reference image with a plurality of second key points extracted from a subsequently obtained image frame. Also referring to FIG. 10, the processor 140 of the X-ray imaging device 100 may obtain a subsequent image frame i₁ by photographing the object with the camera 110, and extract the plurality of second key points P₁, P₂, ..., Pₙ of the landmark of an object 1002 from the subsequent image frame I₁ through inferencing using the Al model 152. The processor 140 may calculate a difference by comparing the plurality of first key points P_{R_1}, P_{R_2}, ..., P_{R_n} extracted from the reference image i_{R} with the plurality of second key points P₁, P₂, ..., Pₙ extracted from the subsequent image frame i₁.

Referring back to FIG. 9, in operation S930, the X-ray imaging device 100 may compare the calculated difference with a preset threshold α.

When the difference exceeds the threshold α as a result of the comparing in operation S940, the X-ray imaging device 100 detects a motion of the object. The processor 140 of the X-ray imaging device 100 may determine that the object has moved when the difference exceeds the threshold α.

When the difference is equal to or less than the threshold α as a result of the comparing in operation S950, the X-ray imaging device 100 does not detect a motion of the object. When no motion is detected, the X-ray imaging device 100 may perform operations of obtaining an image frame (e.g., the second image frame) through subsequent image taking, and proceeding back to operation S920 to extract a plurality of third key points from the image frame (e.g., the second image frame) and calculate a difference by comparing the plurality of extracted third key points with the plurality of first key points P_{R_1}, P_{R_2}, ... , P_{R_n}.

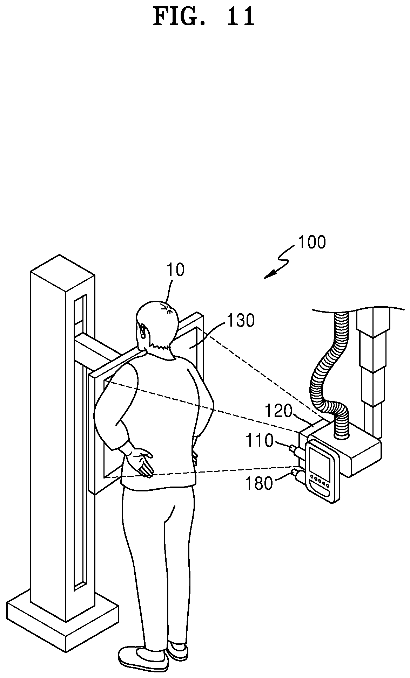

FIG. 11 is a diagram illustrating an operation of the X-ray imaging device 100 for detecting positioning of the object 10 by using a depth measuring device 180, according to an embodiment of the present disclosure.

Referring to FIG. 11, the X-ray imaging device 100 may include the camera 110, the X-ray irradiator 120, the X-ray detector 130 and a depth measuring device 180. The camera 110, the X-ray irradiator 120 and the X-ray detector 130 illustrated in FIG. 11 are the same as the components as described in connection with FIG. 4, so the redundant description will not be repeated.

The depth measuring device 180 is configured to measure a distance between the X-ray irradiator 120 and the object 10. In an embodiment of the present disclosure, the depth measuring device 180 may include at least one of a stereo-type camera, a time of flight (ToF) camera and a laser distance measurer. The processor 140 (see FIG. 5) of the X-ray imaging device 100 may detect patient positioning by measuring the distance between the X-ray irradiator 120 and the object 10, using the depth measuring device 180. After detecting the patient positioning, the processor 140 may perform the motion detection mode in response to a user input (manual mode) being received or after a lapse of a preset time (automatic mode).

FIG. 12 is a block diagram illustrating components of the X-ray imaging device 100 and the workstation 200, according to an embodiment of the present disclosure.

The X-ray imaging device 100 illustrated in FIG. 12 may be implemented in a ceiling type. Referring to FIG. 12, the X-ray imaging device 100 may include the camera 110, the X-ray irradiator 120, the X-ray detector 130, the processor 140, the user input interface 160, the output interface 170 and the communication interface 190. The X-ray imaging device 100 illustrated in FIG. 12 is the same as the X-ray imaging device 100 (see FIG. 5) except that the former does not include the memory 150 (see FIG. 5) but further includes the communication interface 190, so the redundant description will be omitted.

The communication interface 190 may transmit and receive data with the workstation 200 over a wired or wireless communication network and process the data. The communication interface 190 may perform data communication with the workstation 200 by using at least one of data communication schemes including, for example, a wireless local area network (WLAN), Wi-Fi, bluetooth, zigbee, WFD, infrared data association (IrDA), bluetooth low energy (BLE), near field communication (NFC), wireless broadband Internet (Wibro), world interoperability for microwave access (WiMAX), shared wireless access protocol (SWAP), wireless gigabit alliance (WiGig) and radio frequency (RF) communication.

In an embodiment of the present disclosure, the communication interface 190 may transmit the object image obtained by photographing the object through the camera 110 to the workstation 200 and receive a detection result of a motion of the object from the workstation 200, under the control of the processor 140. The X-ray imaging device 100 may display a notification signal (e.g., a graphical UI) that indicates the received detection result of the motion of the object through the display 172 or output it as an acoustic signal through the speaker 174.

The workstation 200 may include the communication interface 210 for communicating with the X-ray imaging device 100, a memory 230 for storing at least one instruction or program code, and the processor 220 configured to execute the instructions or program codes stored in the memory 230. The processor 220 may be a hardware device that makes up the controller 220 (see FIG. 1) as illustrated in FIG. 1.

An Al model 232 may be stored in the memory 230 of the workstation 200. The Al model 232 stored in the workstation 200 is the same as the Al model 152 (see FIGS. 4 and 5) as described and illustrated in FIGS. 4 and 5 except for storage positions, so the redundant description will be omitted. The workstation 200 may receive image data of an object image from the X-ray imaging device 100 through the communication interface 210. In an embodiment of the present disclosure, the image data transmitted to the workstation 200 may include the reference image and the subsequent image frames. The processor 220 of the workstation 200 may detect a motion of the object by comparing the reference image with the subsequent image frames, using the Al model 232.

In an embodiment of the present disclosure, the processor 220 may use a self-organizing map, which is a machine learning algorithm of the Al model 232, to cluster pixels that represent the object and the background, respectively, from each of the reference image and the subsequent image frame, and detect a motion of the object by applying weights to pixels that represent the object. In an embodiment of the present disclosure, the processor 220 may input the reference image and the subsequent image frame to the trained deep neural network model of the Al model 232, extract key points of a landmark of the object from the reference image and the subsequent image frame by performing inferencing using the deep neural network model, and detect a motion of the object by comparing the extracted key points. A specific method by which the processor 220 uses the self-organizing map or the deep neural network model to detect a motion of the object is the same as the operation method of the processor 140 of the X-ray imaging device 100 as described in connection with FIGS. 8 to 10, so the redundant description will not be repeated.

The processor 220 of the workstation 200 may control the communication interface 210 to transmit data of the motion detection result to the X-ray imaging device 100.

In general, the storage capacity of the memory 150 (see FIG. 5) and operation processing speed of the processor 140 of the X-ray imaging device 100 may be restricted as compared to the workstation 200. Hence, the workstation 200 may perform an operation (e.g., detecting a motion of an object through inferencing using the Al model 232) that requires storage of massive data and a huge amount of computation, and then transmit required data (e.g., data of the motion detection result of the object) to the X-ray imaging device 100 over a communication network. In this way, even without a large capacity memory and a processor having a high-speed computation capability, the X-ray imaging device 100 may receive the data of the motion detection result of the object from the workstation 200 and output a notification signal that indicates the motion detection result of the object, thereby reducing the processing time spent on detecting a motion of the object and increasing accuracy in motion detection result.

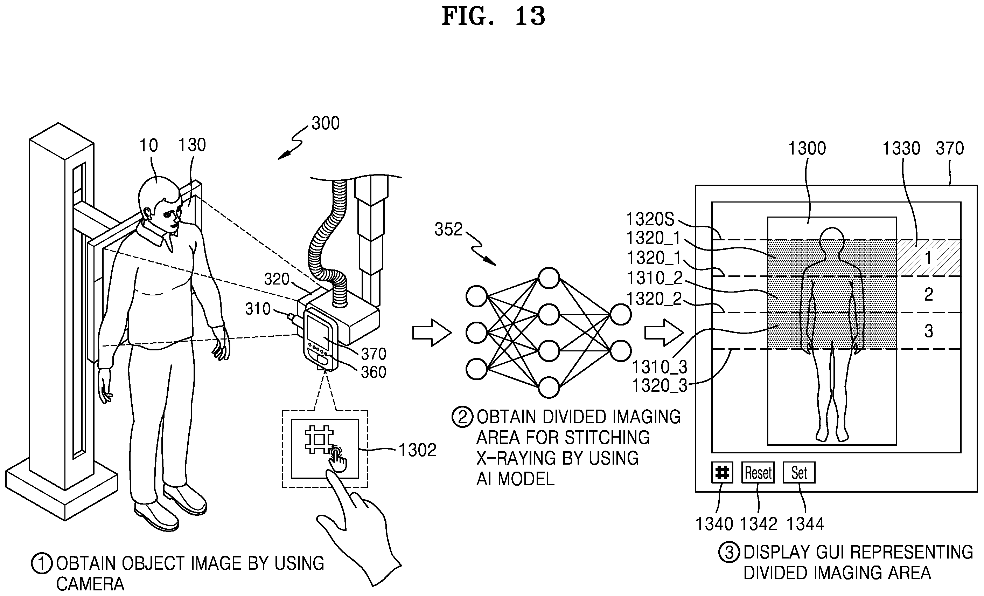

FIG. 13 is a conceptual diagram for describing an operation of an X-ray imaging device 300 for displaying divided imaging areas for stitching X-raying on an image obtained through a camera, according to an embodiment of the present disclosure.

In FIG. 13, the X-ray imaging device 300 is illustrated as a ceiling type, but is not limited thereto. In an embodiment of the present disclosure, the X-ray imaging device 300 may also be implemented in a mobile type.

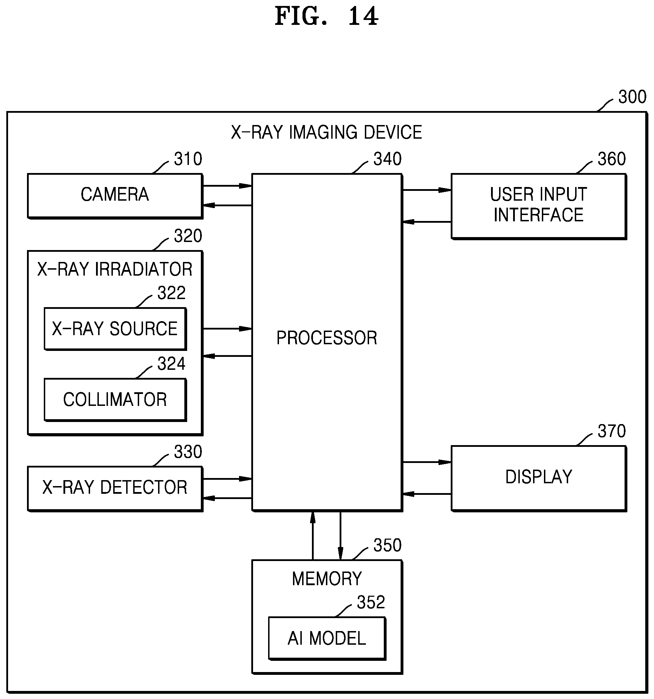

Referring to FIG. 13, the X-ray imaging device 300 may include a camera 310, an X-ray irradiator 320, an X-ray detector 330, a user input interface 360, and a display 370. In FIG. 13, only minimum components for describing the function and/or operation of the X-ray imaging device 300 are illustrated, and components included in the X-ray imaging device 300 are not limited to those illustrated in FIG. 13. The components of the X-ray imaging device 300 will be described in detail in connection with FIG. 14.

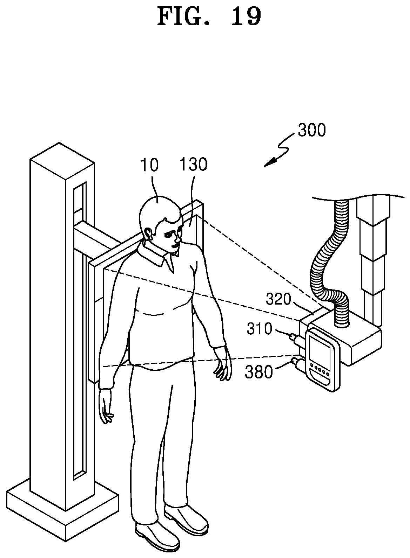

In operation ①, the X-ray imaging device 300 may obtain an object image 1300 by photographing the object 10 with the camera 310. The X-ray imaging device 300 may obtain an image of the object 10 through the camera 310 as patient positioning in front of the X-ray detector 330 is completed. The X-ray imaging device 300 may automatically recognize that the object 10 (e.g., a patient) has been positioned in front of the X-ray detector 330, and in response to the patient positioning being recognized, obtain an image of the object 10 by using the camera 310. In an embodiment of the present disclosure, the X-ray imaging device 300 may further include a depth measuring device 380 (see FIG. 19) implemented with at least one of a stereo-type camera, a ToF camera and a laser distance measurer, and detect the patient positioning by measuring the distance between the X-ray irradiator 320 and the object 10, using the depth measuring device 380.

An object image 1300 obtained in operation ① is a 2D image obtained through the camera 310 having a general image sensor (e.g., CMOS or CCD), which is different from an X-ray image obtained by receiving X-rays transmitted through the object 10 through the X-ray detector 330 and performing image processing on it. In an embodiment of the present disclosure, the X-ray imaging device 300 may display the object image 1300 on the display 370.

The X-ray imaging device 300 obtains, by using the Al model 352, a plurality of divided imaging areas 1310-1 to 1310-3 for stitching X-raying the object 10, in operation ②. The X-ray imaging device 300 may input the object image 1300 to the trained Al model 352, and obtain the plurality of divided imaging areas 1310-1 to 1310-3 for stitching X-raying the object 10 through inferencing using the Al model 352. In an embodiment of the present disclosure, the Al model 352 may be a deep neural network model that is trained by a supervised learning method that applies a plurality of images as input data and applies location coordinates indicating divided imaging areas stitched according to an imaging protocol as the ground truth. The deep neural network model may be a convolutional neural network (CNN) model, but is not limited thereto. The deep neural network model may be implemented with e.g., CenterNet, but is not limited thereto.

In the present disclosure, the term 'stitching' refers to image processing that obtains one X-ray image by connecting the plurality of X-ray images of the plurality of divided X-ray imaging areas 1310-1 to 1310-3. The stitching may include an image process that detects overlapping portions between the X-ray images obtained for the plurality of divided imaging areas 1310-1 to 1310-3 and connects the detected overlapping portions. In FIG. 13, the plurality of divided imaging areas 1310-1 to 1310-3 are illustrated as a total of three obtained by dividing a target imaging area of the object 10, but it is merely illustrative and is not limited thereto. The number of the divided imaging areas 1310-1 to 1310-3 and the number of divisional imaging times may be determined based on at least one of a portion of the object to be X-rayed, an imaging protocol, the size (e.g., height) or the shape (e.g., body type) of the object 10, and may be determined to be two or more.

In an embodiment of the present disclosure, the X-ray imaging device 300 may receive a user input to select an auto stitching planning UI 1302 through the user input interface 360, and in response to the user input being received, obtain the plurality of divided imaging areas 1310-1 to 1310-3 for stitching X-raying from the object image 1300 through the camera 310. In an embodiment of the present disclosure, the auto stitching planning UI 1302 may be a graphical UI displayed on the display 370. In this case, the user input interface 360 and the display 370 may be integrated into a touch screen type.

The X-ray imaging device 300 displays the graphical UI that represents the plurality of divided imaging areas 1310-1 to 1310-3, in operation ③. The X-ray imaging device 300 may display, on the display 370, a plurality of guidelines 1320S, 1320-1, 1320-2 and 1320-3 that represent tops and bottoms of the plurality of divided imaging areas 1310-1 to 1310-3. In an embodiment of the present disclosure, the plurality of guidelines 1320S, 1320-1, 1320-2 and 1320-3 may represent not only the tops and bottoms of the plurality of divided imaging areas 1310-1 to 1310-3 but also left and right boundaries. The X-ray imaging device 300 may display the graphical UI that represents the plurality of guidelines 1320S, 1320-1, 1320-2 and 1320-3 by overlaying them on the plurality of divided imaging areas 1310-1 to 1310-3 in the object image 1300. Among the plurality of guidelines 1320S, 1320-1, 1320-2 and 1320-3 displayed on the display 370, the upper indicator 1320S may be a graphical UI that represents the top of the first divided imaging area 1310-1. The first guideline 1320-1 may be a graphical UI that represents the bottom of the first divided imaging area 1310-1 and represents the top of the second divided imaging area 1310-2, the second guideline 1320-2 may be a graphical UI that represents the bottom of the second divided imaging area 1310-2 and represents the top of the third divided imaging area 1310-3, and the third guideline 1320-3 may be a graphical UI that represents the bottom of the third divided imaging area 1310-3.

In an embodiment of the present disclosure, the X-ray imaging device 300 may display a divided imaging count UI 1330 that represents the number of divided imaging times corresponding to the plurality of divided imaging areas 1310-1 to 1310-3. In FIG. 13, the divided imaging count UI 1330 may display the number of divided imaging times as a number (e.g., 1, 2, 3, ...).

In an embodiment of the present disclosure, the X-ray imaging device 300 may display a graphical UI that includes a stitching icon 1340, a resetting icon 1342 and a setting icon 1344 on the display 370. The stitching icon 1340 is a graphical UI for receiving a user input to display the plurality of guidelines 1320S, 1320-1, 1320-2 and 1320-3 by overlaying them on the object image 1300. The resetting icon 1342 is a graphical UI for receiving a user input to enter a resetting mode for changing at least one of the location, size and shape of the plurality of divided imaging areas 1310-1 to 1310-3 by changing the position of the plurality of guidelines 1320S, 1320-1, 1320-2 and 1320-3. The setting icon 1344 is a graphical UI for receiving a user input to determine the displayed plurality of divided imaging areas 1310-1 to 1310-3 and perform stitching X-raying.

The X-ray imaging device 300 according to an embodiment as illustrated in FIG. 13 may obtain, by using the Al model 352, the plurality of divided imaging areas 1310-1 to 1310-3 for stitching imaging before X-raying and display the plurality of guidelines 1320S, 1320-1, 1320-2 and 1320-3 that represent tops and bottoms of the plurality of divided imaging areas 1310-1 to 1310-3, thereby allowing the user to efficiently, appropriately, conveniently and intuitively understand the number of divided imaging areas. The X-ray imaging device 300 according to an embodiment of the present disclosure may automate the entire stitching procedure, thereby efficiently obtaining X-ray images and reducing the stitching imaging preparation time. Furthermore, in an embodiment of the present disclosure, the X-ray imaging device 300 may provide a technical effect of preventing an increase in radiography time and a risk of extra radiation exposure or overradiation for the patient, which occur in retaking due to inaccurate imaging area settings.

FIG. 14 is a block diagram illustrating components of the X-ray imaging device 300, according to an embodiment of the present disclosure.

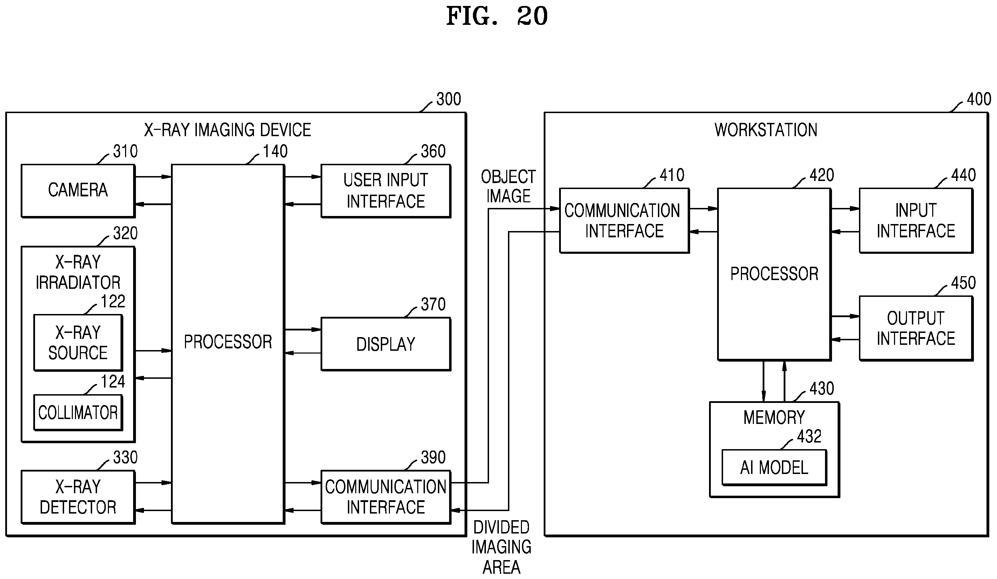

The X-ray imaging device 300 illustrated in FIG. 14 may be a mobile-type device including the mobile X-ray detector 330. It is not, however, limited thereto, and the X-ray imaging device 300 may be implemented in a ceiling type. The X-ray imaging device 300 of the ceiling type will be described in detail later in connection with FIG. 20.

Referring to FIG. 14, the X-ray imaging device 300 may include the camera 310, the X-ray irradiator 320, the X-ray detector 330, the processor 340, the memory 350, the user input interface 360, and the display 370. The camera 310, the X-ray irradiator 320, the X-ray detector 330, the processor 340, the memory 350, the user input interface 360, and the display 370 may be electrically and/or physically connected to one another. In FIG. 14, only essential components for describing operations of the X-ray imaging device 300 are illustrated, and components included in the X-ray imaging device 300 are not limited to those illustrated in FIG. 14. In an embodiment of the present disclosure, the X-ray imaging device 300 may further include a communication interface 390 (see FIG. 20) for performing data communication with the workstation 400 (see FIG. 20), the server 2000 (see FIG. 1), the other medical device 3000 (see FIG. 1) or the external portable terminal 4000 (see FIG. 1).

The camera 310, the X-ray irradiator 320 and the X-ray detector 330 are the same components as the camera 110 (see FIG. 5), the X-ray irradiator 120 (see FIG. 5) and the X-ray detector 130 (see FIG. 5) described and illustrated in FIG. 5, and perform the same functions and/or operations of the camera 110, the X-ray irradiator 120 and the X-ray detector 130, so the redundant descriptions will not be repeated.

The processor 340 may execute one or more instructions of a program stored in the memory 350. The processor 340 may include hardware components for performing arithmetic, logical, and input/output operations and image processing. The processor 340 is illustrated as one element in FIG. 14, but is not limited thereto. In an embodiment of the present disclosure, the processor 340 may be configured with one or more elements. The processor 340 may be a universal processor such as a central processing unit (CPU), an application processor (AP), a digital signal processor (DSP), etc., a dedicated graphic processor such as a graphic processing unit (GPU), a vision processing unit (VPU), etc., or a dedicated artificial intelligence (AI) processor such as a neural processing unit (NPU). The processor 340 may control processing of input data according to a predefined operation rule or an Al model. When the processor 340 is the dedicated Al processor, the dedicated Al processor may be designed in a hardware structure specialized for processing with a particular Al model.

The memory 350 may include, for example, at least one type of storage media including a flash memory, a hard disk, a multimedia card micro type memory, a card type memory (e.g., SD or XD memory), a random access memory (RAM), a static random access memory (SRAM), a read-only memory (ROM), an electrically erasable programmable read-only memory (EEPROM), a programmable read-only memory (PROM), or an optical disk.

The memory 350 may store instructions related to functions and/or operations of the X-ray imaging device 300 for obtaining divided imaging areas for stitching X-raying from an object image obtained by the camera 310 and displaying the plurality of guideline Uls that represent tops, bottoms and left and right boundaries of the divided imaging areas. In an embodiment of the present disclosure, the memory 350 may store at least one of algorithms, data structures, program codes, application programs, and instructions that are readable to the processor 340. The instructions, algorithms, data structures and program codes stored in the memory 350 may be implemented in e.g., a programming or scripting language such as C, C++, Java, assembler, etc.

In the following embodiments, the processor 340 may be implemented by executing the instructions or program codes stored in the memory 350.

The processor 340 may obtain image data of the object image obtained by photographing the object from the camera 310. In response to patient positioning in front of the X-ray detector 330 being completed, the processor 340 may obtain the image data of the object by controlling the camera 310 to obtain images of the object. In an embodiment of the present disclosure, the processor 340 may receive the user's touch input that selects a button UI for performing an automatic stitching imaging mode through the user input interface 360, and control the camera 310 to obtain images of the object for stitching imaging in response to the touch input being received. The user input to perform the automatic stitching imaging mode is not limited to the touch input, but may correspond to an input that presses a key pad, a hardware button, a jog switch, etc.

It is not, however, limited thereto, and the X-ray imaging device 300 may further include the depth measuring device 380 (see FIG. 19), and the processor 340 may recognize the object positioned in front of the X-ray detector 330 by using the depth measuring device 380, and perform the automatic stitching imaging mode to automatically obtain images of the object in response to the object being recognized. A specific embodiment in which the processor 340 recognizes positioning of the object by using the depth measuring device 380 will be described in FIG. 19 in detail.

The processor 340 may obtain the plurality of divided imaging areas for stitching X-raying through inferencing that analyzes the object image using an Al model 352. In an embodiment of the present disclosure, the Al model 352 may be implemented with the instructions, program codes or algorithms stored in the memory 350, but is not limited thereto. In an embodiment of the present disclosure, the Al model 352 may not be included in the X-ray imaging device 300. In this case, an Al model 432 (see FIG. 20) may be included in the workstation 400 (see FIG. 20).

The processor 340 may input the object image to the Al model 352, and obtain the plurality of divided imaging areas for stitching X-raying through inferencing using the Al model 352. In an embodiment of the present disclosure, the Al model 352 may be a deep neural network model that is trained by a supervised learning method that applies the obtained plurality of images of the object as input data and applies divided imaging areas stitched according to an imaging protocol as the ground truth. The ground truth of the divided imaging area may be differently determined depending on the imaging protocol. To increase accuracy of the divided imaging area, the deep neural network model may be trained in a partially modified form. When the input data (e.g., a plurality of images) and ground truth data (e.g., location coordinates of the divided imaging areas) for training are insufficient, it is also possible to process the data by augmentation to increase an amount of the data or apply a fine tuning method that partially modifies the trained model.

In an embodiment of the present disclosure, the deep neural network model may be a convolutional neural network (CNN) model. The deep neural network model may be implemented with, for example, CenterNet. It is not, however, limited thereto, and the deep neural network model may be implemented with, for example, recurrent neural networks, restricted Boltzmann machines, deep Belief networks, bidirectional recurrent deep neural networks or deep Q-networks.

The size of the plurality of divided imaging areas obtained through the Al model 352 may be larger than the size of an area available to be obtained by the X-ray detector 330. In an embodiment of the present disclosure, the processor 340 may adjust the size of the plurality of divided imaging areas obtained through the Al model 352 to be smaller than the size of the X-ray detector 330.

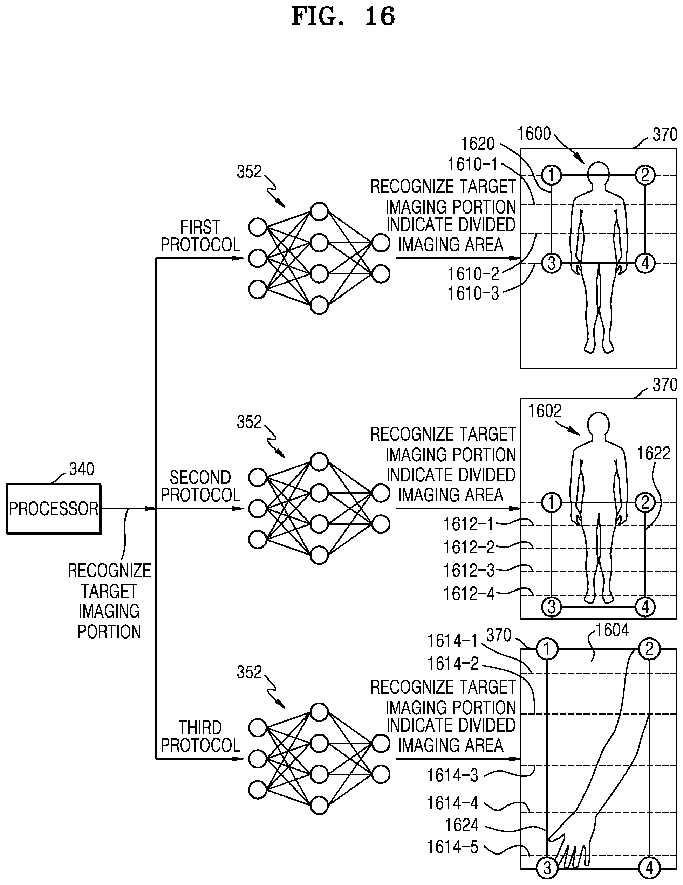

The processor 340 may recognize a target imaging portion from the object image based on an imaging protocol, input information about the recognized target imaging portion to the Al model 352 along with the object image, and obtain a plurality of divided imaging areas through inferencing using the Al model 352. A specific embodiment in which the processor 340 obtains the plurality of divided imaging areas based on the target imaging portion will be described in FIG. 16 in detail.

The processor 340 may display the object image through the display 370. In an embodiment of the present disclosure, the processor 340 may control the display 370 to display the graphical UI that represents the plurality of divided imaging areas by overlaying it on the object image.

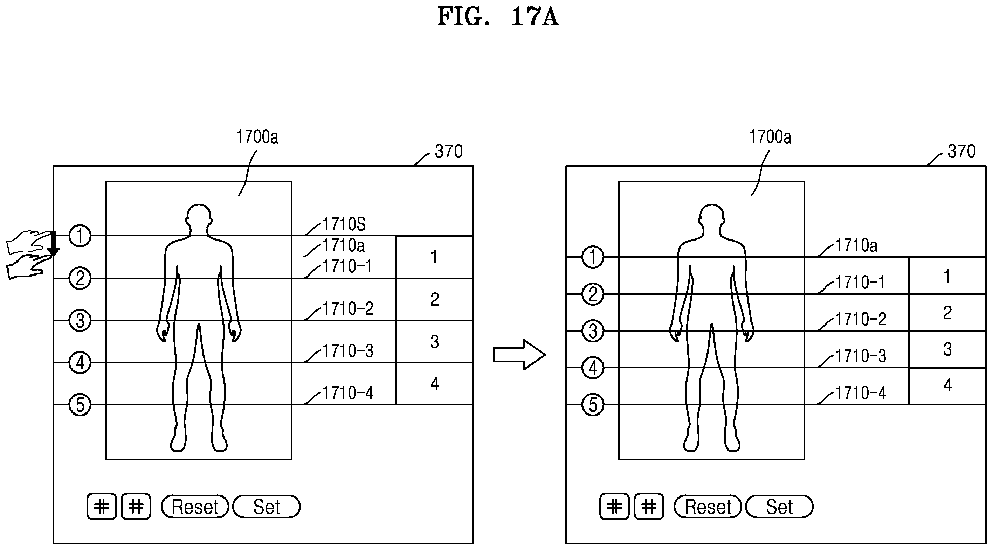

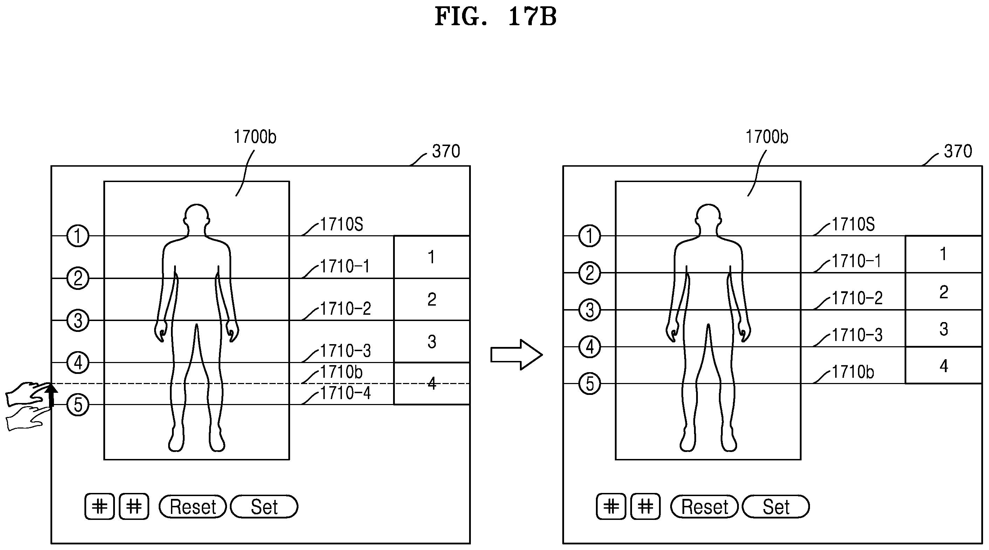

The user input interface 360 may receive a user input to adjust a location of at least one of the plurality of guidelines that represent tops, bottoms and left and right boundaries of the plurality of divided imaging areas. In an embodiment of the present disclosure, the user input interface 360 may receive the user's touch input to adjust the location of at least one of the plurality of guidelines. The processor 340 may change at least one of the position, size and shape of the plurality of divided imaging areas by adjusting the location of at least one of the plurality of guidelines based on the user input received through the user input interface 360. A specific embodiment in which the processor 340 changes at least one of the location, size and shape of the plurality of divided imaging areas based on the user input will be described in detail in connection with FIGS. 17A and 17B.

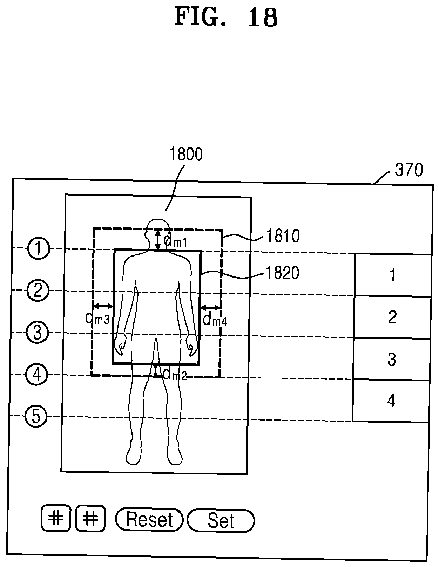

The user input interface 360 may receive a user input to adjust the size of a margin between an X-ray imaging area and the target imaging area by adjusting the size of the target imaging area of the object. The processor 340 may determine top, bottom, left and right margin sizes of the plurality of divided imaging areas based on the user input received through the user input interface 360. A specific embodiment in which the processor 340 determines or adjusts the margin size of the X-ray imaging area based on a user input will be descried in detail in connection with FIG. 18.

The processor 340 may obtain at least one divided X-raying image by X-raying a plurality of divided imaging areas. The processor 340 may control the X-ray irradiator 320 to irradiate X-rays onto the object, receive, through the X-ray detector, X-rays transmitted through the object, and obtain a plurality of divided X-raying images by converting the received X-rays to electric signals. The processor 340 may obtain an X-ray image of a target X-raying area by stitching the plurality of divided X-raying images.

The user input interface 360 is configured to provide an interface for operating the X-ray imaging device 300. The user input interface 360 may be made up with, for example, but not exclusively, a control panel including hardware elements such as a keypad, a mouse, a track ball, a jog dial, a jog switch or a touch pad. In an embodiment of the present disclosure, the user input interface 360 may be configured as a touch screen that receives a touch input and displays a graphical UI.

The display 370 may display the object image under the control of the processor 340. In an embodiment of the present disclosure, the display 370 may display the graphical UI that represents the plurality of divided imaging areas by overlaying it on the object image under the control of the processor 340. The display 370 may be configured with a hardware device including at least one of e.g., a CRT display, an LCD display, a PDP display, an OLED display, an FED display, an LED display, a VFD display, a DLP display, a flat panel display, a 3D display, and a transparent display, but is not limited thereto. In an embodiment of the present disclosure, the display 370 may include a touch screen having a touch interface. In a case that the display 370 is configured as a touch screen, the display 370 may be a component integrated with the user input interface 360 comprised of a touch panel.

Although not illustrated in FIG. 14, the X-ray imaging device 300 may further include a speaker configured to output an acoustic signal. In an embodiment of the present disclosure, the processor 340 may control the speaker to output information relating to completion of setting the plurality of divided imaging areas in a voice or notification sound.

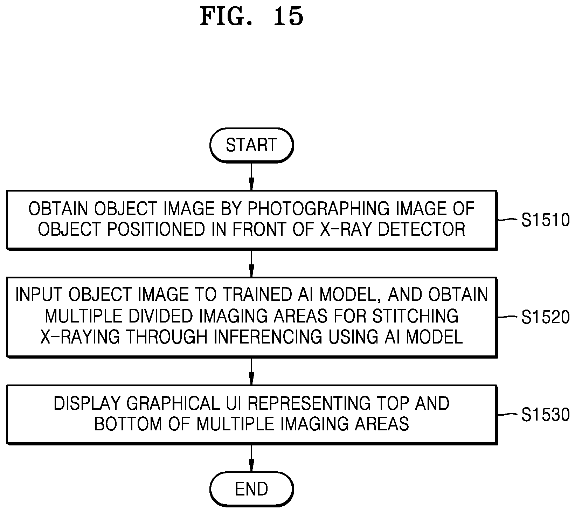

FIG. 15 is a flowchart illustrating a method by which the X-ray imaging device 300 obtains divided imaging areas for stitching X-raying on an image obtained through a camera and displaying a graphical user interface (UI) representing the divided imaging areas, according to an embodiment of the present disclosure.

In operation S1510, the X-ray imaging device 300 obtains an object image by photographing the object positioned in front of the X-ray detector. The X-ray imaging device 300 may obtain image data by using the camera to photograph the object (e.g., a patient) positioned in front of the X-ray detector 330 (see FIGS. 13 and 14). The object image 1300 obtained in operation 1510 is a 2D image obtained through the camera having a general image sensor (e.g., CMOS or CCD), which is different from an X-ray image obtained by receiving, through the X-ray detector 330, X-rays transmitted through the object and performing image processing on it.

In operation S1520, the X-ray imaging device 300 inputs the object image to a trained Al model, and obtains a plurality of divided imaging areas for stitching X-raying through inferencing using the Al model. In an embodiment of the present disclosure, the Al model may be a deep neural network model that is trained by a supervised learning method that applies the obtained plurality of images of the object as input data and applies divided imaging areas stitched according to an imaging protocol as the ground truth. The deep neural network model may be a convolutional neural network (CNN) model. The deep neural network model may be implemented with, for example, CenterNet. It is not, however, limited thereto, and the deep neural network model may be implemented with, for example, recurrent neural networks, restricted Boltzmann machines, deep Belief networks, bidirectional recurrent deep neural networks or deep Q-networks.

The X-ray imaging device 300 may recognize a target imaging portion from the object image based on an imaging protocol, and obtain a plurality of divided imaging areas through inferencing that inputs information about the recognized target imaging portion to the Al model along with the object image.

In operation S1530, the X-ray imaging device 300 displays a graphical UI that represents tops, bottoms and left and right boundaries of the plurality of divided imaging areas. In an embodiment of the present disclosure, the X-ray imaging device 300 may display the object image, and display the plurality of guidelines that represent tops, bottoms and left and right boundaries of the plurality of divided imaging areas by overlaying them on the object image.

In an embodiment of the present disclosure, the X-ray imaging device 300 may output a voice or notification sound that provides the user with information relating to completion of setting the plurality of divided imaging areas.

The X-ray imaging device 300 may obtain a plurality of divided X-raying images by X-raying the plurality of divided imaging areas, and obtain an X-ray image of a target X-raying area by stitching the obtained plurality of divided X-raying images.

FIG. 16 is a diagram illustrating an operation of the X-ray imaging device 300 for determining divided imaging areas according to an imaging protocol and displaying a graphical UI representing the determined divided imaging areas, according to an embodiment of the present disclosure.

The processor 340 of the X-ray imaging device 300 may recognize the imaging protocol from the object image. The imaging protocol may include, for example, a whole spine protocol, a long bone protocol, or an extremity protocol, but is not limited thereto. The processor 340 may recognize a target imaging portion from the object image based on the imaging protocol. For example, in the case of the whole spine protocol, the processor 340 may recognize, from the object image, portions from ears to below the pelvis, e.g., head, shoulders, elbows, hands, waist, etc., as the target imaging portion. For example, in the case of the long bone protocol, the processor 340 may recognize portions from waist to toes as the target imaging portion, and in the case of the extremity protocol, the processor 340 may recognize a portion such as face, hands or feet as the target imaging portion.

The processor 340 may input information about the recognized target imaging portion to the Al model 352 along with the object image, and perform inferencing using the Al model 352. The Al model 352 may be a deep neural network model that is trained by a supervised learning method that applies the plurality of images of the object as input data and applies divided imaging areas stitched according to an imaging protocol as the ground truth. The ground truth of the divided imaging area may be differently determined depending on the imaging protocol. For example, in a case of the whole spine protocol among imaging protocols, head, shoulders, elbows, hands, waist, etc., among the body portions from ears to below the pelvis, may be determined as divided areas, and in a case of the long bone protocol, a portion from waist to toes may be determined as divided areas. For example, in a case of the extremity protocol for head (skull), hands or feet, portions with unique body characteristics such as the face, hands, or feet may be determined as divided areas. The processor 340 may recognize a target imaging portion from the object image through inferencing using the deep neural network model, and display the divided imaging areas.

In the process of inferencing using the deep neural network model, when data of the deep neural network model is encrypted, the processor 340 may decode the data. In a case that a plurality of candidates of the target imaging area are output as a result of inferencing of the deep neural network model, the processor 340 may determine a final target imaging area by selecting one of the plurality of candidates having the highest confidence.

Referring to the embodiment illustrated in FIG. 16, the processor 340 may recognize a first protocol from a first object image 1600. The processor 340 may input the first object image 1600 and the recognized first protocol to the Al model 352, recognize a first target imaging area 1620 through inferencing using the Al model 352, and obtain a plurality of guidelines 1610-1 to 1610-3 that divide the first target imaging area 1620 into a plurality of divided imaging areas. For example, the first protocol may be the whole spine protocol, and the first target imaging area 1620 may include a portion from ears to below the pelvis among body portions. The plurality of guidelines 1610-1 to 1610-3 may be a graphical UI that indicates tops and bottoms of the plurality of divided areas of the head, the shoulder, the elbow, the waist, etc., among the portion from ears to below the pelvis. The processor 340 may display the first object image 1600 on the display 370, and display the first target imaging area 1620 and the plurality of guidelines 1610-1 to 1610-3 by overlaying them on the first object image 1600.

Likewise, the processor 340 may recognize a second protocol from a second object image 1602, recognize a second target imaging area 1622 corresponding to the second protocol from the second object image 1602 through inferencing using the Al model 352, and obtain a plurality of guidelines 1612-1 to 1612-4 that divide the second target imaging area 1622 into a plurality of divided imaging areas. For example, the second protocol may be the long bone protocol, and the second target imaging area 1622 may include a portion from waist to toes among body portions. The processor 340 may display the second target imaging area 1622 and the plurality of guidelines 1612-1 to 1612-4 by overlaying them on the second object image 1602 displayed on the display 370. The processor 340 may recognize a third protocol from a third object image 1604, recognize a third target imaging area 1624 corresponding to the third protocol from the third object image 1604 through inferencing using the Al model 352, and obtain a plurality of guidelines 1614-1 to 1614-5 that divide the third target imaging area 1624 into a plurality of divided imaging areas. For example, the third protocol may be the extremity protocol, and the third target imaging area 1624 may include a portion from shoulders to fingertips among body portions. The processor 340 may display the third target imaging area 1624 and the plurality of guidelines 1614-1 to 1614-5 by overlaying them on the third object image 1604 displayed on the display 370.

In the embodiment illustrated in FIG. 16, the X-ray imaging device 300 may recognize a target imaging portion for each imaging protocol, and as target imaging areas 1620, 1622 and 1624 for the respective imaging protocols are recognized through inferencing using the Al model 352, the X-ray imaging device 300 may automate stitching X-raying according to the imaging protocol and detect accurate and reliable target imaging areas 1620, 1622 and 1624. Accordingly, user convenience may be increased, and time for stitching X-ray imaging may be reduced.

FIG. 17A is a diagram illustrating an operation of the X-ray imaging device 300 for changing at least one of location, size and shape of a divided imaging area based on a user input, according to an embodiment of the present disclosure.

Referring to FIG. 17A, an object image 1700a may be displayed on the display 370, and a plurality of guidelines 1710S, and 1710-1 to 1710-4 that represent tops and bottoms of the plurality of divided imaging areas may be displayed on the object image 1700a. The user may adjust the location of at least one of the plurality of guidelines 1710S, and 1710-1 to 1710-4. The user input interface 360 (see FIG. 14) of the X-ray imaging device 300 may receive a user input to adjust the location of at least one of the plurality of guidelines 1710S and 1710-1 to 1710-4. In an embodiment of the present disclosure, the user input interface 360 may be configured with a touch screen including a touch pad, in which case, the user input interface 360 may be a component integrated with the display 370. The user input interface 360 may receive the user's touch input to adjust the location of at least one of the plurality of guidelines 1710S and 1710-1 to 1710-4. It is not, however, limited thereto, and the user input interface 360 may receive a user input to adjust the location of at least one of the plurality of guidelines 1710S and 1710-1 to 1710-4 through a key pad, a hardware button, a mouse, a jog switch or a jog dial. In the embodiment illustrated in FIG. 17A, the user input interface 360 may receive a user input to adjust the location of the upper indicator 1710S downward, which represents the top of the first divided imaging area among the plurality of guidelines 1710S and 1710-1 to 1710-4.

In response to the user input being received, the processor 340 (see FIG. 14) of the X-ray imaging device 300 may change the location of at least one guideline, and change at least one of the location, size and shape of the plurality of divided imaging areas based on the at least one changed guideline. In the embodiment illustrated in FIG. 17A, the processor 340 may change the size and shape of the plurality of divided imaging areas based on an upper indicator 1710a whose location is adjusted by the user input. The processor 340 may change the size and location of the plurality of divided imaging areas by evenly dividing an area between the upper indicator 1710a whose location is adjusted and the fourth guide line 1710-4. The processor 340 may display the changed upper indicator 1710a and the plurality of changed divided imaging areas on the display 370.

In an embodiment of the present disclosure, as the location of the at least one guideline is adjusted by the user input, the processor 340 may change the number of divided imaging times. For example, in a case that a user input to adjust the location of the upper indicator 1710S downward is received, the processor 340 may reduce the number of divided imaging times. For example, in a case that a user input to adjust the location of the upper indicator 1710S upward or adjust the location of the fourth guideline 1710-4 downward is received, the processor 340 may increase the number of divided imaging times. For example, the processor 340 may reduce the number of divided imaging times from four to three, and evenly divide an area between the upper indicator 1710a whose location is adjusted by the user input and the fourth guideline 1710-4 into three areas.

It is not, however, limited thereto, and in an embodiment of the present disclosure, the processor 340 may unevenly divide the area between the upper indicator 1710a and the fourth guideline 1710-4. For example, as the location of the upper indicator 1710a is adjusted, the processor 340 may change the size and shape of only the first divided imaging area.

FIG. 17B is a diagram illustrating an operation of the X-ray imaging device 300 for changing at least one of location, size and shape of a divided imaging area based on a user input, according to an embodiment of the present disclosure.

FIG. 17B is the same as FIG. 17A except that one of the plurality of guidelines 1710S and 1710-1 to 1710-4 that is adjusted by the user input is the third guideline 1710-3 and that the location of the third guideline 1710-3 is adjusted upward by the user input, so the overlapping description will be omitted.

Referring to FIG. 17B, the user input interface 360 (see FIG. 14) of the X-ray imaging device 300 may receive a user input to adjust the location of the fourth guideline 1710-4 upward, which represents the bottom of the fourth divided imaging area among the plurality of guidelines 1710S and 1710-1 to 1710-4. In response to the user input being received, the processor 340 (see FIG. 14) of the X-ray imaging device 300 may change the size and shape of the plurality of divided imaging areas based on the fourth guideline 1710b whose location is adjusted by the user input. The processor 340 may change the size and location of the plurality of divided imaging areas by evenly dividing an area between the first guideline 1710-1 and the fourth guideline 1710b based on the fourth guideline 1710b whose location is adjusted.

It is not, however, limited thereto, and the processor 340 may unevenly divide the area between the first guideline 1710-1 and the fourth guideline 1710b. For example, the processor 340 may change the size and shape of only the fourth divided imaging area as the location of the third guideline 1710b is adjusted upward.

In an embodiment illustrated in FIGS. 17A and 17B, the X-ray imaging device 300 may change at least one of the location, size and shape of the plurality of divided imaging areas obtained by the Al model 352 (see FIGS. 13 and 14) by adjusting the location of the plurality of guidelines 1710S and 1710-1 to 1710-4 by the user input. Accordingly, in a special case that a divided imaging area is inappropriately obtained by the Al model 352 or the user needs to change or adjust the divided imaging area in person, the X-ray imaging device 300 according to an embodiment of the present disclosure may allow the user to manually adjust the location, size and shape of the plurality of divided imaging areas, thereby increasing user convenience and enabling accurate X-ray imaging.

FIG. 18 is a diagram illustrating an operation of the X-ray imaging device 300 for determining a margin of a divided imaging area based on a user input, according to an embodiment of the present disclosure.

Referring to FIG. 18, the X-ray imaging device 300 may display an object image 1800 on the display 370, and display a target imaging area 1810, which is a graphical UI representing X-ray imaging, by overlaying it on the object image 1800. The user input interface 360 (see FIG. 14) of the X-ray imaging device 300 may receive a user input to determine an X-raying area 1820 by adjusting one of a plurality of margins dₘ₁ to dₘ₄ in up, down, left and right directions. In an embodiment of the present disclosure, the user input interface 360 may be configured as a touch screen including a touch pad, in which case, the user input interface 360 may be a component integrated with the display 370. The user input interface 360 may receive the user's touch input to adjust one of the plurality of margins dₘ₁ to dₘ₄ in the up, down, left and right directions of the target imaging area 1810, which is a graphical UI, displayed on the touch screen. It is not, however, limited thereto, and the user input interface 360 may receive a user input to adjust one of the plurality of margins dₘ₁ to dₘ₄ in the up, down, left and right directions through a key pad, a hardware button, a mouse, a jog switch or a jog dial.

The processor 340 (see FIG. 14) of the X-ray imaging device 300 may adjust the size of one of the plurality of margins dₘ₁ to dₘ₄ in the up, down, left and right directions based on the user input received through the user input interface 360. The processor 340 may set the X-raying area 1820 based on the margin whose size is adjusted.

FIG. 19 is a diagram illustrating an operation of the X-ray imaging device 300 for detecting positioning of the object 10 by using a depth measuring device 380, according to an embodiment of the present disclosure.

Referring to FIG. 19, the X-ray imaging device 300 may include the camera 310, the X-ray irradiator 320, the X-ray detector 330 and the depth measuring device 380. The camera 310, the X-ray irradiator 320 and the X-ray detector 330 illustrated in FIG. 19 are the same as the camera 110 (see FIG. 4), the X-ray irradiator 120 (see FIG. 4) and the X-ray detector 130 (see FIG. 4), so the redundant description will not be repeated.

The depth measuring device 380 is configured to measure a distance between the X-ray irradiator 320 and the object 10. In an embodiment of the present disclosure, the depth measuring device 380 may include at least one of a stereo-type camera, a time of flight (ToF) camera and a laser distance measurer. The processor 340 (see FIG. 14) of the X-ray imaging device 300 may detect patient positioning by measuring the distance between the X-ray irradiator 320 and the object 10, using the depth measuring device 380. After detecting the patient positioning, the processor 340 may obtain an object image by photographing the object 10 through the camera 310, and obtain a plurality of divided imaging areas by analyzing the object image, using the Al model 352 (see FIGS. 13 and 14).

In an embodiment of the present disclosure, in a case that the distance between the X-ray irradiator 320 and the object 10 obtained through the depth measuring device 380, i.e., a source to image distance (SID), is out of a preset range, the processor 340 may determine that the object 10 is abnormally positioned in an imaging location. In this case, the processor 340 may output divided imaging areas set by default regardless of the imaging protocol on the display 370 (see FIGS. 13 and 14).

FIG. 20 is a block diagram illustrating components of the X-ray imaging device 300 and the workstation 400, according to an embodiment of the present disclosure.

The X-ray imaging device 300 illustrated in FIG. 20 may be implemented in a ceiling type. Referring to FIG. 20, the X-ray imaging device 300 may include the camera 310, the X-ray irradiator 320, the X-ray detector 330, the processor 340, the user input interface 360, the display 370 and the communication interface 390. The X-ray imaging device 300 illustrated in FIG. 20 is the same as the X-ray imaging device 300 (see FIG. 14) except that the former does not include the memory 350 (see FIG. 14) but may further include the communication interface 390, so the redundant description will be omitted.

The communication interface 390 may process data while transmitting and receiving data with the workstation 400 over a wired or wireless communication network. The communication interface 390 may perform data communication with the workstation 400 by using at least one of data communication schemes including, for example, a wireless local area network (WLAN), Wi-Fi, bluetooth, zigbee, WFD, infrared data association (IrDA), bluetooth low energy (BLE), near field communication (NFC), wireless broadband Internet (Wibro), world interoperability for microwave access (WiMAX), shared wireless access protocol (SWAP), wireless gigabit alliance (WiGig) and radio frequency (RF) communication.

In an embodiment of the present disclosure, the communication interface 390 may transmit an object image obtained by photographing the object through the camera 310 to the workstation 400 and receive data about the divided imaging areas for stitching X-raying from the workstation 400, under the control of the processor 340. The X-ray imaging device 300 may display a plurality of guidelines that represent the divided imaging areas on the object image through the display 370, based on the received data about the divided imaging areas.

The workstation 400 may include the communication interface 410 for communicating with the X-ray imaging device 300, a memory 430 for storing at least one instruction or program code, and the processor 420 configured to execute the instructions or program codes stored in the memory 430.

An Al model 432 may be stored in the memory 430 of the workstation 400. The Al model 432 stored in the workstation 400 is the same as the Al model 352 (see FIGS. 13 and 14) as described and illustrated in FIGS. 13 and 14 except for storage positions, so the redundant description will be omitted. The workstation 400 may receive image data of an object image from the X-ray imaging device 300 through the communication interface 410. The processor 420 of the workstation 400 may input the object image to the Al model 432, and obtain the plurality of divided imaging areas for stitching X-raying through inferencing using the Al model 432. In an embodiment of the present disclosure, the workstation 400 may receive information regarding an imaging protocol from the X-ray imaging device 300 through the communication interface 410 or receive a user input to set an imaging protocol through the input interface 440. The processor 420 may recognize a target imaging portion from the object image based on the imaging protocol, input information about the recognized target imaging portion to the Al model 432 along with the object image, and obtain a plurality of divided imaging areas through inferencing using the Al model 432.

The processor 420 of the workstation 400 may control the communication interface 410 to transmit data about the divided imaging areas to the X-ray imaging device 300.

In general, the storage capacity of the memory 350 (see FIG. 14) and operation processing speed of the processor 340 of the X-ray imaging device 300 may be restricted as compared to the workstation 400. Hence, the workstation 400 may perform an operation (e.g., obtaining the divided imaging areas through inferencing using the Al model 432) that requires storage of massive data and a huge amount of computation, and then transmit required data (e.g., data about the divided imaging areas) to the X-ray imaging device 300 over a communication network. In this way, even without a large capacity memory and a processor having a high-speed computation capability, the X-ray imaging device 300 may receive the data about the divided imaging areas from the workstation 400 and display a plurality of guidelines that represent the divided imaging areas, thereby reducing the processing time spent on obtaining the divided imaging area and increasing accuracy in the divided imaging area.

The present disclosure provides an X-ray imaging device 100 for detecting a motion of an object. In an embodiment of the present disclosure, the X-ray imaging device 100 may include the X-ray irradiator 120 configured to generate and irradiate X-rays onto an object, the X-ray detector 130 configured to detect X-rays irradiated by the X-ray irradiator 120 and transmitted through the object, the camera 110 configured to obtain an object image by photographing an image of the object positioned in front of the X-ray detector 130, the display 172 and at least one processor 140. The at least one processor 140 may be configured to detect a motion of the object from the object image by analyzing the object image by using an Al model using an Al model. The at least one processor 140 may be configured to output a notification signal on the display 172 to notify a user of a result of the detecting of the motion of the object.

In an embodiment of the present disclosure, the at least one processor 140 may be configured to obtain a reference image by photographing an image of the object that completes positioning in front of the X-ray detector 130, and obtain an image frame by taking a subsequent image of the object after obtaining the reference image. The at least one processor 140 may detect a motion of the object by comparing the object recognized from the reference image with the object recognized from the image frame through the Al model based analysis.

In an embodiment of the present disclosure, the at least one processor 140 may use a self-organizing map among Al models to obtain weights for pixels representing the object recognized from the reference image. The at least one processor 140 may use the weights to detect a motion of the object by comparing the object recognized from the reference image with the object recognized from the image frame. The at least one processor 140 may use a result of the detecting to update the reference image and the weights.

In an embodiment of the present disclosure, the at least one processor 140 may extract a plurality of first key points for a landmark of the object from the reference image through inferencing using a trained deep neural network model among Al models. The at least one processor 140 may calculate a difference between key points by comparing the extracted plurality of first key points with a plurality of second key points of the object extracted from the image frame. The at least one processor 140 may detect a motion of the object by comparing the calculated difference with a preset threshold.

In an embodiment of the present disclosure, the deep neural network model may be a model trained by a supervised learning method that applies a plurality of obtained images as input data and applies location coordinates of key points of the landmark as ground truth.

In an embodiment of the present disclosure, the X-ray imaging device 100 may further include a user input interface configured to receive a user input for selecting a motion detection mode after patient positioning is completed. The at least one processor 140 may perform the motion detection mode based on the received user input, and detect a motion of the object in response to the motion detection mode being performed.

In an embodiment of the present disclosure, the at least one processor 140 may perform the motion detection mode after a lapse of a preset time after the patient positioning is completed. The at least one processor 140 may detect a motion of the object in response to the motion detection mode being performed.

In an embodiment of the present disclosure, the X-ray imaging device 100 may further include the depth measuring device 180 including at least one of a stereo-type camera, a time of flight (ToF) camera and a laser distance measurer. The at least one processor 140 may detect patient positioning by using the depth measuring device 180 to measure the distance between the X-ray irradiator 120 and the object]

In an embodiment of the present disclosure, the at least one processor 140 may set motion detection sensitivity based on at least one of a source to image distance (SID), which is a distance between the object and the X-ray irradiator 120, the size and shape of the object, and an imaging protocol.

In an embodiment of the present disclosure, the display 172 may display a graphical UI having a preset color that represents a motion of the object.

In an embodiment of the present disclosure, the X-ray imaging device 100 may further include a speaker 174 configured to output at least one acoustic signal among a voice and a notification sound that notifies the user of information about a motion of the object.

The present disclosure provides a method of operating the X-ray imaging device 100. According to an embodiment of the present disclosure, a method of operating the X-ray imaging device 100 may include obtaining image data of an object by photographing an image of the object with the camera 110 (S610). According to an embodiment of the present disclosure, the method of operating the X-ray imaging device 100 may include detecting a motion of the object from the image data (S620) by analyzing the image data using an Al model. According to an embodiment of the present disclosure, the method of operating the X-ray imaging device 100 may include outputting a notification signal to notify a user of a result of the detecting of the motion of the object (S630).

In an embodiment of the present disclosure, the obtaining of the image data (S610) may include obtaining a reference image by photographing the object which completes positioning in front of the X-ray detector 130 using the camera 110, and obtaining an image frame by taking a subsequent image of the object after obtaining the reference image. The detecting of the motion of the object (S620) may include detecting a motion of the object by comparing the object recognized from the reference image with the object recognized from the image frame through the Al model based analysis.

In an embodiment of the present disclosure, the detecting of the motion of the object (S620) may include obtaining weights from the reference image by using a self-organizing map (S810), and using the weights to detect a motion of the object by comparing the object recognized from the image frame with the object recognized from the reference image (S820). The detecting of the motion of the object (S620) may include updating the reference image and the weights by using a result of the detecting (S830).

In an embodiment of the present disclosure, the detecting of the motion of the object (S620) may include extracting a plurality of first key points of a landmark of the object from the reference image through inferencing using a trained deep neural network model (S910), calculating a difference between key points by comparing the extracted plurality of first key points with a plurality of second key points of the object extracted from the image frame (S920), and detecting a motion of the object by comparing the calculated difference with a preset threshold.

In an embodiment of the present disclosure, the method of operating the X-ray imaging device 100 may further include receiving a user input to select a motion detection mode after patient positioning is completed. The detecting of the motion of the object (S620) may include performing a motion detection mode based on a user input, and detecting a motion of the object in response to the motion detection mode being performed.

In an embodiment of the present disclosure, the method of operating the X-ray imaging device 100 may further include performing the motion detection mode after a lapse of a preset time after the patient positioning is completed. The detecting of the motion of the object (S620) may include detecting a motion of the object in response to the motion detection mode being performed.

In an embodiment of the present disclosure, the method of operating the X-ray imaging device 100 may further include setting motion detection sensitivity based on at least one of a source to image distance (SID), which is a distance between the object and the X-ray irradiator 120, the size and shape of the object, and an imaging protocol.

In an embodiment of the present disclosure, the outputting of the notification signal (S630) may include displaying a graphical UI having a preset color that represents a motion of the object.

In an embodiment of the present disclosure, the method of operating the X-ray imaging device 100 may include outputting at least one acoustic signal among a voice and a notification sound that notifies the user of information about a motion of the object.

The present disclosure provides the X-ray imaging device 300 for performing stitching X-raying. In an embodiment of the present disclosure, the X-ray imaging device 300 may include the X-ray detector 330 for detecting X-rays irradiated by the X-ray irradiator 320 and transmitted through the object, the camera 310 for obtaining an object image by photographing the object positioned in front of the X-ray detector 330, the display 370 and at least one processor 340. The at least one processor 340 may be configured to input the object image to a trained Al model, and obtain a plurality of divided imaging areas for stitching X-raying the object through inferencing using an Al model. The at least one processor 340 may be configured to display a plurality of guidelines on the display 370 to indicate top, bottom, and left and right boundaries of each of the plurality of divided imaging areas.

In an embodiment of the present disclosure, the Al model may be a deep neural network model that is trained by a supervised learning method that applies the obtained plurality of images as input data and applies divided imaging areas stitched according to an imaging protocol as the ground truth.

In an embodiment of the present disclosure, the at least one processor 340 may recognize a target imaging portion from the object image based on an imaging protocol. The at least one processor 340 may input information about the recognized target imaging portion to the Al model along with the object image, and obtain a plurality of divided imaging areas through inferencing using the Al model.

In an embodiment of the present disclosure, the at least one processor 340 may adjust the size of the plurality of divided imaging areas to be smaller than the size of the X-ray detector 330.

In an embodiment of the present disclosure, the X-ray imaging device 300 may further include a user input interface 360 configured to receive a user input adjusting a location of at least one of the plurality of guidelines. The at least one processor 340 may change at least one of the location, size and shape of the plurality of divided imaging areas by adjusting the location of at least one of the plurality of guidelines based on the received user input.

In an embodiment of the present disclosure, the at least one processor 340 may determine up, down, left and right margin sizes of the plurality of divided imaging areas based on margin information set by a user input.

In an embodiment of the present disclosure, the at least one processor 340 may control the display 370 to display a graphical UI that represents the plurality of divided imaging areas by overlaying it on the object image.

In an embodiment of the present disclosure, the X-ray imaging device 300 may further include a speaker for outputting information relating to completion of setting the plurality of divided imaging areas in a voice or notification sound.

In an embodiment of the present disclosure, the X-ray imaging device 300 may further include the depth measuring device 380 including at least one of a stereo-type camera, a time of flight (ToF) camera and a laser distance measurer. The at least one processor 340 may detect object positioning in front of the X-ray detector 330 by measuring a distance between the X-ray irradiator 320 and the object using the depth measuring device 330.

In an embodiment of the present disclosure, the at least one processor 340 may obtain a plurality of divided X-raying images by X-raying the plurality of divided imaging areas. The at least one processor 340 may obtain an X-ray image of a target X-raying area by stitching the plurality of divided X-raying images.

The present disclosure provides a computer program product including a computer-readable storage medium. The storage medium may include instructions readable to the X-ray imaging device 100 to perform obtaining an object image by photographing an image of an object with a camera; detecting a motion of the object from the object image by analyzing the object image using an Al model; and outputting a notification signal notifying the user of a result of the detecting the motion of the object.

A program executed by the X-ray imaging device 100 as described in the present disclosure may be implemented in hardware elements, software elements, and/or a combination thereof. The program may be performed by any system capable of performing computer-readable instructions.

The software may include a computer program, codes, instructions, or one or more combinations of them, and may configure a processing device to operate as desired or instruct the processing device independently or collectively.

The software may be implemented with a computer program including instructions stored in a computer-readable recording (or storage) medium. Examples of the computer-readable recording medium include a magnetic storage medium (e.g., a read only memory (ROM), a floppy disk, a hard disk, etc.), and an optical recording medium (e.g., a compact disc ROM (CD-ROM), or a digital versatile disc (DVD)). The computer-readable recording medium may also be distributed over network coupled computer systems so that the computer-readable code is stored and executed in a distributed fashion. The media may be read by the computer, stored in the memory, and executed by the processor.

The computer-readable storage medium may be provided in the form of a non-transitory storage medium. The term 'non-transitory' only means that the storage medium is tangible without including a signal, but does not help distinguish any data stored semi-permanently or temporarily in the storage medium. For example, the non-transitory storage medium may include a buffer that temporarily stores data.

Furthermore, the program according to the disclosed embodiments of the present specification may be provided in a computer program product. The computer program product may be a commercial product that may be traded between a seller and a buyer.

The computer program product may include a software program and a computer-readable storage medium having the software program stored thereon. For example, the computer program product may include a product (e.g., a downloadable application) in the form of a software program that is electronically distributed by the manufacturer of the X-ray imaging device or by an electronic market (e.g., Samsung Galaxy store^{®}). For the electronic distribution, at least a portion of the software program may be stored in a storage medium or arbitrarily created. In this case, the storage medium may be one of a server of the manufacturer of the X-ray imaging device 100 or of a relay server that temporarily stores the software program.

The computer program product may include a storage medium of a server or a storage medium of the X-ray imaging device 100 in a system including the X-ray imaging device 100 and/or the server. Alternatively, in a case that there is a third device (e.g., a workstation 200 (see FIG. 12)) communicatively connected to the X-ray imaging device 100, the computer program product may include a storage medium of the third device. In another example, the computer program product may be transmitted from the X-ray imaging device 100 to the third device, or may include a software program itself that is transmitted from the third device to the electronic device.

In this case, one of the X-ray imaging device 100 or the third device (e.g., the workstation 200 (see FIG. 12)) may execute the computer program product to perform the method according to the disclosed embodiments. Alternatively, at least one of the X-ray imaging device 100 and the third device may execute the computer program product to perform the method according to the disclosed embodiments in a distributed fashion.

For example, the X-ray imaging device 100 may execute the computer program product stored in the memory 150 (see FIG. 5) to control another electronic device communicatively connected to the X-ray imaging device 100 to perform the method according to the disclosed embodiments.

In another example, the third device may execute the computer program product to control the electronic device communicatively connected to the third device to perform the method according to the disclosed embodiments.

In the case that the third device executes the computer program product, the third device may download the computer program product from the X-ray imaging device 100 and execute the downloaded computer program product. Alternatively, the third device may execute the computer program product that is preloaded to perform the method according to the disclosed embodiments.

Although the present disclosure is described with reference to some embodiments as described above and accompanying drawings, it will be apparent to those of ordinary skill in the art that various modifications and changes can be made to the embodiments. For example, the aforementioned method may be performed in a different order, and/or the aforementioned components such as a computer system or a module may be combined in a different form from what is described above, and/or replaced or substituted by other components or equivalents thereof, to obtain appropriate results.

## Claims

1. An X-ray imaging device (100) for detecting a motion of an object, the X-ray imaging device (100) comprising:
an X-ray irradiator (120) configured to generate X-rays and irradiate the X-rays to the object;
an X-ray detector (130) configured to detect X-rays irradiated by the X-ray irradiator (120) and transmitted through the object;
a camera (110) configured to obtain an object image by photographing the object positioned in front of the X-ray detector (130);
a display (172); and
at least one processor (140) configured to: detect a motion of the object from the object image by analyzing the object using an artificial intelligence (AI) model, and output a notification signal notifying a user of a result of the detecting of the motion of the object on the display (172).

2. The X-ray imaging device (100) of claim 1, wherein the at least one processor (140) is configured to:
obtain a reference image by photographing the object with the camera (110), the object completing positioning in front of the X-ray detector (130),
obtain an image frame by subsequently photographing the object after obtaining the reference image, and
detect a motion of the object by comparing the object recognized from the reference image with the object recognized from the image frame through analysis using the Al model.

3. The X-ray imaging device (100) of claim 2, wherein the at least one processor (140) is configured to:
obtain weights for pixels representing the object recognized from the reference image by using a self-organizing map of the Al model,
detect a motion of the object by comparing the object recognized from the image frame with the object recognized from the reference image by using the weights, and
update the reference image and the weights by using a result of the detecting.

4. The X-ray imaging device (100) of claim 2, wherein the at least one processor (140) is configured to:
extract a plurality of first key points of a landmark of the object from the reference image through inferencing using a trained deep neural network model of the Al model,
calculate a difference between key points by comparing the extracted plurality of first key points with a plurality of second key points of the object extracted from the image frame, and
detect a motion of the object by comparing the calculated difference with a preset threshold.

5. The X-ray imaging device (100) of claim 4, wherein the deep neural network model is a model trained by supervised learning method which applies a plurality of obtained images as input data and applies location coordinates of key points of the landmark as ground truth.

6. The X-ray imaging device (100) of any one of claims 1 to 5, further comprising:
a depth measuring device (180) including at least one of a stereo-type camera, a time of flight (ToF) camera and a laser distance measurer,
wherein the at least one processor (140) is configured to:
detect patient positioning by measuring a distance between the X-ray irradiator (120) and the object by using the depth measuring device (180).

7. The X-ray imaging device (100) of any one of claims 1 to 6, wherein the at least one processor (140) is configured to:
set motion detection sensitivity based on at least one of a source to image distance (SID), which is a distance between the object and the X-ray irradiator (120), a size and a shape of the object, and an imaging protocol.

8. The X-ray imaging device (100) of any of claims 1 to 7, wherein the display (172) displays a graphical user interface (UI) having a preset color representing a motion of the object.

9. The X-ray imaging device (100) of any one of claims 1 to 8, further comprising:
a speaker (174) configured to output at least one acoustic signal among a voice and a notification sound notifying the user of information about a motion of the object.

10. A method of operating an X-ray imaging device (100), the method comprising:
obtaining image data of an object by photographing the object with a camera (110) (S610);
detecting a motion of the object from the image data by analyzing the image data using an artificial intelligence (AI) model (S620); and
outputting a notification signal notifying a user of a result of the detecting of the motion of the object (S630).

11. The method of claim 10, wherein the obtaining of the image data (S610) comprises:
obtaining a reference image by photographing the object using the camera (110), the object completing positioning in front of an X-ray detector (130); and
obtaining an image frame by subsequently photographing the object after obtaining the reference image,
the detecting of the motion of the object comprises detecting a motion of the object by comparing the object recognized from the reference image with the object recognized from the image frame through analysis using the Al model.

12. The method of claim 11, wherein the detecting of the motion of the object (S620) comprises:
obtaining weights from the reference image by using a self-organizing map (S810);
detecting a motion of the object by comparing the object recognized from the image frame with the object recognized from the reference image by using the weights (S820); and
updating the reference image and the weights by using a result of the detecting (S830).

13. The method of claim 11, wherein the detecting of the motion of the object (S620) comprises:
extracting a plurality of first key points of a landmark of the object from the reference image through inferencing using a trained deep neural network model (S910);
calculating a difference between key points by comparing the extracted plurality of first key points with a plurality of second key points of the object extracted from the image frame (S920); and
detecting a motion of the object by comparing the calculated difference with a preset threshold.

14. The method of any one of claims 10 to 13, further comprising:
setting motion detection sensitivity based on at least one of a source to image distance (SID), which is a distance between the object and the X-ray irradiator (120), a size and a shape of the object, and an imaging protocol.

15. The method of any one of claims 10 to 14, wherein the outputting of the notification signal (S630) comprises displaying a graphical user interface (UI) having a preset color representing a motion of the object.
